# FASCICULE DE BREVET EUROPEEN

(11) **EP 3 189 075 B1**
(45) Date de publication et mention de la délivrance du brevet: **12.12.2018**
(21) Numéro de dépôt: 15759793.1
(22) Date de dépôt: 04.09.2015
(51) Int. Cl.: C07K 16/06, C07K 1/36, C07K 1/18, C07K 1/22, C07K 1/34

(54) **PROCÉDÉ DE PURIFICATION D'UN ANTICORPS MONOCLONAL**
VERFAHREN ZUR REINIGUNG VON MONOKLONALEN ANTIKÖRPERN
METHOD FOR PURIFICATION OF MONOCLONAL ANTIBODIES

(30) Priorité: 05.09.2014 FR 1458346
(43) Date de publication de la demande: 12.07.2017
(73) Titulaire: Laboratoire Français du Fractionnement et des Biotechnologies, 91940 Les Ulis (FR)
(72) Inventeur: COUTARD, François, F-30100 Ales (FR)
(74) Mandataire: Regimbeau
(86) Numéro de dépôt international: PCT/EP2015/070298
(87) Numéro de publication internationale: WO 2016/034726

(56) Documents cités:
- WO-A1-2013/009526
- WO-A1-2013/013193
- WO-A2-2011/050071
- WO-A2-2013/177115
- HUI F. LIU ET AL: "Recovery and purification process development for monoclonal antibody production", MABS, vol. 2, no. 5, 1 septembre 2010 (2010-09-01), pages 480-499, XP055027612, ISSN: 1942-0862, DOI: 10.4161/mabs.2.5.12645 cité dans la demande
- FAHRNER R L ET AL: "INDUSTRIAL PURIFICATION OF PHARMACEUTICAL ANTIBODIES: DEVELOPMENT, OPERATION, AND VALIDATION OF CHROMATOGRAPHY PROCESSES", BIOTECHNOLOGY AND GENETIC ENGINEERING REVIEWS, INTERCEPT LTD., ANDOVER, GB, vol. 18, 1 juillet 2001 (2001-07-01), pages 301-327, XP008034714, ISSN: 0264-8725 cité dans la demande

## Description

### DOMAINE DE L'INVENTION

La présente invention se situe dans le domaine des procédés de purification d'anticorps monoclonaux ou de protéines de fusion Fc destinés à des applications pharmaceutiques. Elle concerne un procédé de purification d'un anticorps monoclonal ou d'une protéine de fusion entre le fragment Fc d'un anticorps et un second polypeptide, comprenant a) une étape de chromatographie d'affinité sur une résine ayant pour matrice un gel de polymère de méthacrylate réticulé, sur lequel est greffée de la protéine A, b) une étape d'inactivation virale, c) une étape de chromatographie échangeuse de cations sur une résine ayant pour matrice un gel d'agarose réticulé, sur lequel sont greffés des groupements sulfonate (-SO₃-) par l'intermédiaire de bras espaceurs à base de dextrane, d) une étape de chromatographie échangeuse d'anions sur une membrane hydrophile de polyéthersulfone revêtue d'un polymère réticulé sur lequel sont greffés des groupements amine quaternaire (Q), et e) une étape de nanofiltration avec un filtre ayant une double membrane de polyéthersulfone d'une porosité d'environ 20 nm, caractérisé en ce que le tampon d'élution utilisé à l'étape a) pour éluer l'anticorps est un tampon formiate utilisé à une molarité de 5 à 10 mM et à un pH compris entre 2,6 et 3,6.

### ART ANTERIEUR

On a assisté au cours de la dernière décennie à un fort développement des traitements d'immunothérapie passive à l'aide d'anticorps, souvent monoclonaux, dans différents domaines thérapeutiques : cancers, prévention de l'alloimmunisation chez les femmes enceintes Rhésus-négatives, maladies infectieuses, maladies inflammatoires et notamment autoimmunes.

Afin de pouvoir être utilisé comme médicament, un anticorps doit répondre à des exigences stringentes en termes de qualité, de pureté, et de sécurité sanitaire. Par conséquent, différents procédés de purification d'anticorps ont été développés afin de satisfaire ces exigences. Ces procédés impliquent généralement plusieurs étapes de purification par chromatographie, ainsi qu'une ou plusieurs étapes d'élimination ou d'inactivation virale (Fahrner et al. Biotechnol Genet Eng Rev. 2001;18:301-27 ; Liu et al. MAbs. 2010 Sep-Oct;2(5):480-99).

Bien que de nombreux procédés permettant d'obtenir des anticorps purifiés satisfaisant les exigences des autorités de santé aient été décrits, il existe néanmoins un besoin pour des procédés de purification optimisés. En effet, les procédés existants imposent aux fabricants des coûts significatifs de production, qu'il est important de réduire au maximum de façon à diminuer le coût des traitements par anticorps monoclonaux.

Le coût global d'un procédé de production d'anticorps varie en fonction d'un certain nombre de facteurs, tels que notamment le coût de chacun des produits utilisés pour la purification, la quantité d'anticorps susceptible d'être traitée en une seule fois, la durée de mise en oeuvre de chacune des étapes du procédé, et le rendement de chacune des étapes du procédé.

De plus, ces facteurs sont interdépendants, c'est-à-dire que le choix d'un produit particulier pour une des étapes de purification va impacter différemment chacun de ces facteurs.

Or, pour chacune des étapes de purification susceptibles d'être utilisées dans un procédé de purification d'un anticorps monoclonal, l'homme du métier doit réaliser un choix entre de nombreux produits disponibles commercialement, chacun ayant ses avantages et ses inconvénients. Ainsi, dans le cadre d'une étape de purification par chromatographie, l'homme du métier doit faire des choix au niveau de la résine de chromatographie (base et ligand) et des tampons utilisés. Or, il existe de très nombreuses résines de chromatographies, fondées sur différentes bases (agarose, dextrane, polymères synthétiques, etc...) et différents ligands (affinité, échange de cations, échange d'ions, interactions hydrophobes, etc...), et plusieurs tampons sont susceptibles d'être utilisés pour chaque résine de chromatographie.

Il est donc particulièrement difficile pour l'homme du métier de sélectionner une combinaison d'étapes et de produits spécifiques capables de réduire significativement les coûts de purification d'un anticorps monoclonal, tout en maintenant le niveau de qualité, de pureté, et de sécurité sanitaire de l'anticorps purifié.

### RESUME DE L'INVENTION

Dans le cadre de la présente invention, les inventeurs ont mis en évidence qu'une combinaison particulière d'étapes de purification et d'élimination ou d'inactivation virale permet de diminuer d'un facteur 3 les coûts de purification d'un anticorps monoclonal, tout en maintenant le niveau de qualité, de pureté, et de sécurité sanitaire de l'anticorps purifié. Cette forte diminution du coût de purification d'un anticorps monoclonal a été permise par la sélection de produits moins coûteux, permettant de traiter une quantité d'anticorps plus élevé en une seule fois, et par l'optimisation des conditions opératoires de chaque étape afin de maintenir le niveau de qualité, de pureté, et de sécurité sanitaire de l'anticorps purifié.

Dans un premier aspect, la présente invention concerne donc un procédé de purification d'un anticorps monoclonal ou d'une protéine de fusion entre le fragment Fc d'un anticorps et un second polypeptide, tel que revendiqué dans les revendications 1 à 10. Plus généralement, la présente description divulgue un procédé de purification d'un anticorps monoclonal ou d'une protéine de fusion entre le fragment Fc d'un anticorps et un second polypeptide, comprenant :
a) une étape de chromatographie d'affinité sur une résine ayant pour matrice un gel de polymère de méthacrylate réticulé, sur lequel est greffée de la protéine A,
b) une étape d'inactivation virale,
c) une étape de chromatographie échangeuse de cations sur une résine ayant pour matrice un gel d'agarose réticulé, sur lequel sont greffés des groupements sulfonate (-SO₃-) par l'intermédiaire de bras espaceurs à base de dextrane,
d) une étape de chromatographie échangeuse d'anions sur une membrane hydrophile de polyéthersulfone revêtue d'un polymère réticulé sur lequel sont greffés des groupements amine quaternaire (Q), et
e) une étape de nanofiltration avec un filtre ayant une double membrane de polyéthersulfone d'une porosité d'environ 20 nm.

Avantageusement, le gel de polymère de méthacrylate réticulé sur lequel est greffée de la protéine A utilisé à l'étape a) est sous forme de billes ayant un diamètre moyen compris entre 30 et 60 µm, avantageusement entre 40 et 50 µm. Dans l'invention, le tampon d'élution utilisé à l'étape a) pour éluer l'anticorps est un tampon formiate, celui-ci étant utilisé à une molarité de 5 à 10 mM et à un pH compris entre 2,6 et 3,6. Avantageusement, l'étape b) est réalisée par incubation pendant 30 à 120 minutes à une température de 20 à 25°C dans un milieu comprenant 0,5 à 2% (v/v) de polyoxyéthylène-p-t-octylphénol (Triton X-100, N° CAS 9002-93-1).

Avantageusement, le tampon utilisé lors de l'étape d) est un tampon trishydroxyméthylaminométhane (TRIS) à une concentration de 15 à 25 mM, un pH de 7,5 à 8,5 et une conductivité de 5 à 15 mS/cm.

Avantageusement, l'étape e) comprend en outre une filtration préalable à travers un pré-filtre VPF (Viresolve PreFilter, filtre en profondeur comprenant des fibres de cellulose, de la terre de diatomée et une résine chargée négativement) ou Viresolve pro Shield (membrane de polyéthersulfone d'une porosité de 0,22 µm fonctionnalisée par des groupements SO₃⁻).

Avantageusement, le procédé comprend en outre une étape d'ultrafiltration et/ou de diafiltration.

Avantageusement, le procédé selon l'invention est mis en oeuvre sur un surnageant de culture d'un clone producteur de l'anticorps monoclonal ou de la protéine de fusion entre le fragment Fc d'un anticorps et un second polypeptide.

Avantageusement, le procédé selon l'invention est mis en oeuvre pour la purification d'un anticorps monoclonal, en particulier d'un anticorps dirigé contre l'un des antigènes suivants : Rhésus D, CD2, CD3, CD4, CD19, CD20, CD22, CD25, CD30, CD33, CD40, CD51 (Integrin alpha-V), CD52, CD80, CTLA-4 (CD152), SLAMF7 (CD319), Her2/neu, EGFR, EPCAM, CCR4, CEA, FR-alpha, GD2, GD3, HLA-DR, IGF1R (CD221), phosphatidylserine, TRAIL-R1, TRAIL-R2, antigènes de *Clostridium difficile*, antigènes de *Staphylococcus aureus* (notamment ClfA et acide lipotheicoïque), antigènes du cytomégalovirus (notamment la glycoprotéine B), antigènes d'*Escherichia coli* (notamment toxine Shiga-like, sous unité IIB), antigènes du virus respiratoire syncytial (Protéine F notamment), antigènes du virus de l'hépatite B, antigènes du virus Influenza A (Hémagglutinine notamment), antigènes de *Pseudomonas aeruginosa* sérotype IATS O11, antigènes du virus de la rage (Glycoprotéine notamment), ou phosphatidylserine.

### DESCRIPTION DES FIGURES

**Figure 1****.** Détermination du volume injecté au point 10%BT pour une purification par chromatographie d'affinité sur Protéine A sur des résines de type MabSelect SuRe™ (A), Poros GoPure™(B), Toyopearl AF-rProtein A-650F (C) et Amsphere™ Protein A JWT203 (D).

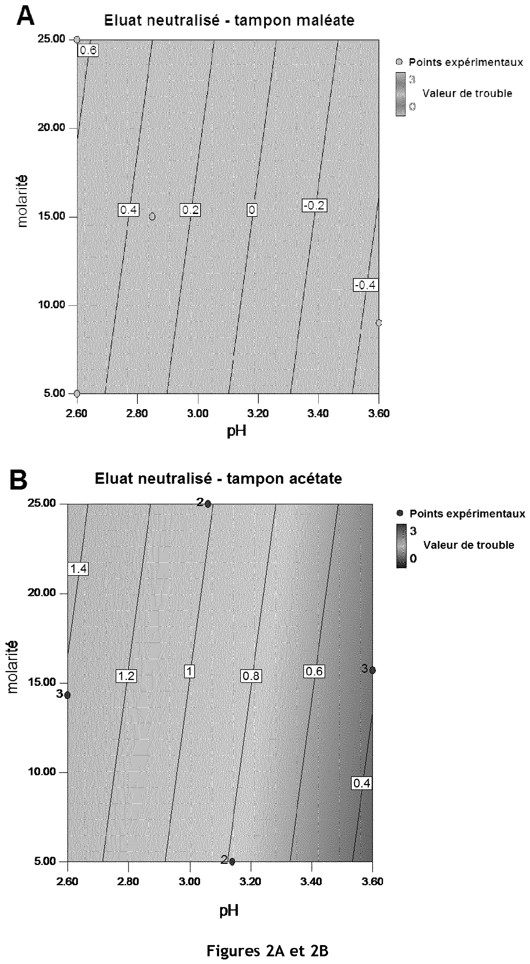

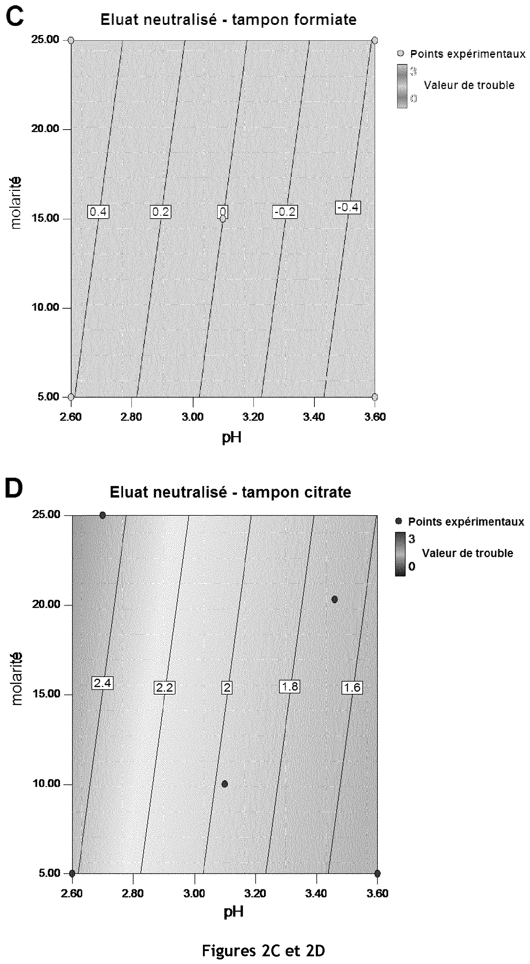

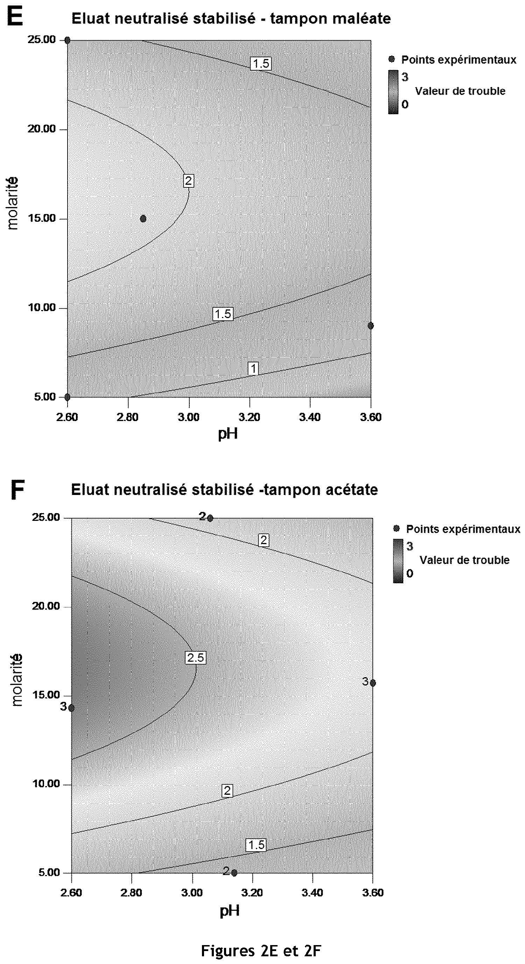

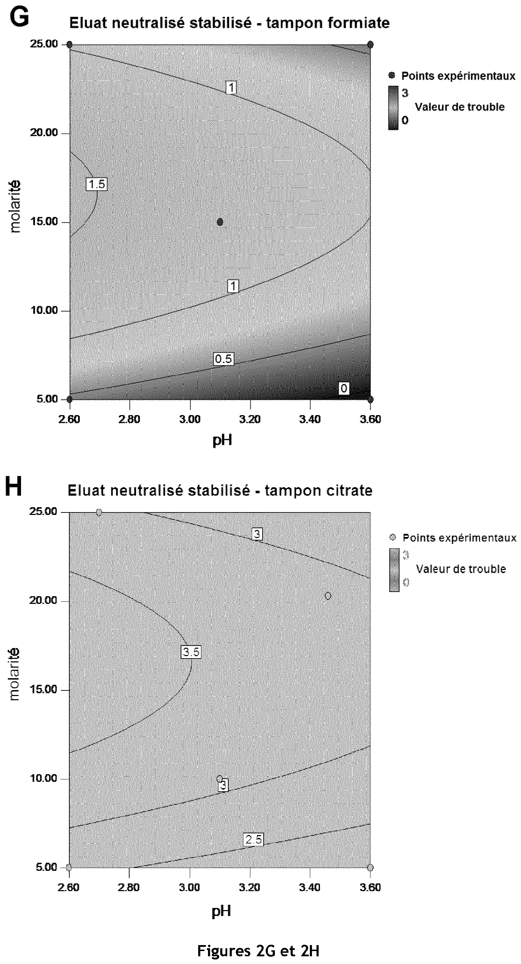

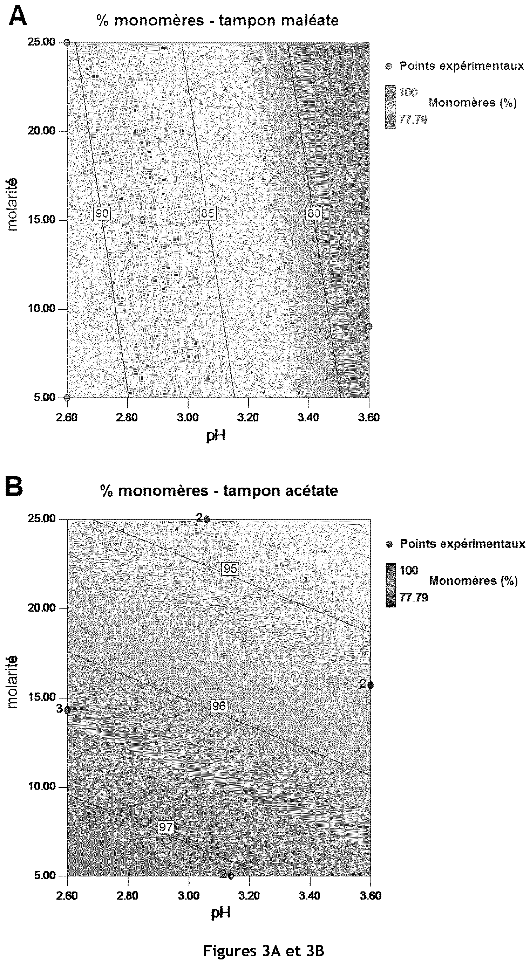

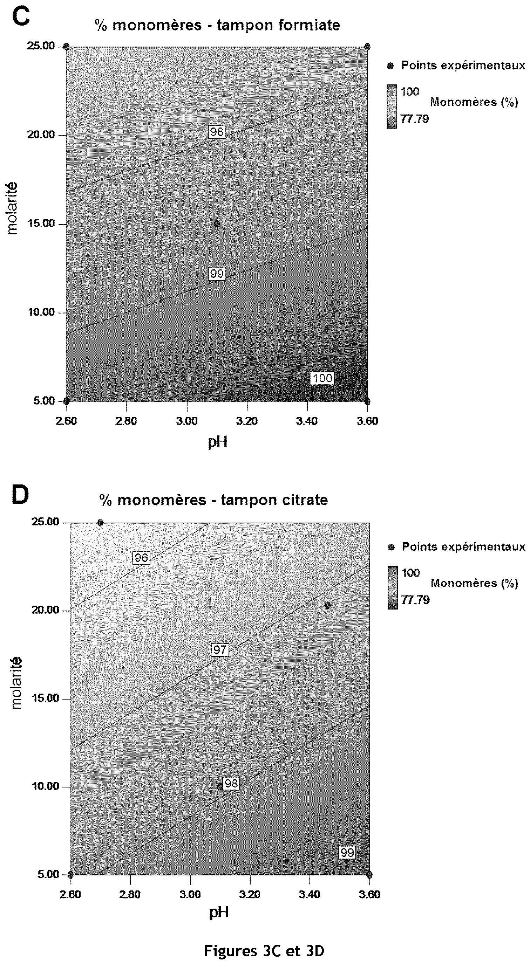

**Figure 2****.** Analyse par le logiciel Design-Expert® Software du trouble des éluats neutralisés (juste après neutralisation, A à D) et des éluats neutralisés stabilisés (1 heure après neutralisation, E à H) suite à une élution de la colonne de protéine A par un tampon maléate (A et E), acétate (B et F), formiate (C et G) ou citrate (D et H), en fonction du pH (représenté en abscisse) et de la molarité (représentée en ordonnée) du tampon. Pour un couple (pH/molarité) donné, le trouble de chaque éluat est noté par une valeur variant entre 0 et 3, une valeur de 0 correspondant à un éluat limpide et une valeur de 3 à un éluat fortement troublé (opalescent). Les courbes représentant les couples (pH/molarité) correspondant à une valeur de trouble donnée sont représentées. **Figure 3****.** Analyse du pourcentage de monomères dans des éluats neutralisés suite à une élution de la colonne de protéine A par un tampon maléate (A), acétate (B), formiate (C) ou citrate (D), en fonction du pH (représenté en abscisse) et de la molarité (représentée en ordonnée) du tampon. Les courbes représentant les couples (pH/molarité) correspondant à une valeur de pourcentage de formes monomériques de l'anticorps dans l'éluat neutralisé sont représentées.
**Figure 4****.** Débit de filtration (g/h/m²) en fonction de la charge d'anticorps (g/m²) pour une nanofiltration sur un filtre Planova® 15N (A), Planova® 20N (B), ou Viresolve® Pro 20N (C).
**Figure 5****.** Charge d'anticorps (g/m²) nanofiltrée en fonction du temps de filtration (minutes) pour une nanofiltration sur un filtre Planova® 15N, Planova® 20N, ou Viresolve® Pro 20N.
**Figure 6****.** Variation du rendement moyen en fonction du pH d'élution utilisé pour la colonne A3-JSR.
**Figure 7****.** Variation de l'élimination moyenne des HCP en fonction du pH d'élution utilisé pour la colonne A3-JSR.
**Figure 8****.** Variation de l'élimination moyenne des HCP en fonction de la concentration en NaCl dans la solution de lavage pour la colonne A3-JSR.

### DESCRIPTION DETAILLEE DE L'INVENTION

Comme indiqué précédemment, les procédés de purification d'anticorps monoclonaux imposent aux fabricants des coûts significatifs de production, qu'il est important de réduire au maximum de façon à diminuer le coût des traitements par anticorps monoclonaux. Or, l'existence de nombreux produits commerciaux distincts susceptibles d'être utilisés pour la purification d'anticorps monoclonaux et la possibilité très importante de variations des conditions opératoires de chaque étape rend la sélection d'une combinaison d'étapes, de produits et de conditions opératoires appropriés pour réduire le coût global de purification extrêmement difficile pour l'homme du métier. Pourtant, les inventeurs ont maintenant mis en évidence qu'une combinaison particulière d'étapes de purification et d'élimination ou d'inactivation virale permet de diminuer d'un facteur 3 les coûts de purification d'un anticorps monoclonal, tout en maintenant le niveau de qualité, de pureté, et de sécurité sanitaire de l'anticorps purifié. Cette forte diminution du coût de purification d'un anticorps monoclonal a été permise par la sélection de produits moins coûteux, de produits permettant de traiter une quantité d'anticorps plus élevé en une seule fois, et par l'optimisation des conditions opératoires de chaque étape afin de maintenir le niveau de qualité, de pureté, et de sécurité sanitaire de l'anticorps purifié.

La présente invention concerne donc un procédé de purification d'un anticorps monoclonal ou d'une protéine de fusion entre le fragment Fc d'un anticorps et un second polypeptide, tel que revendiqué dans les revendications 1 à 10.
Plus généralement, la présente description divulgue un procédé de purification d'un anticorps monoclonal ou d'une protéine de fusion entre le fragment Fc d'un anticorps et un second polypeptide, comprenant :
a) une étape de chromatographie d'affinité sur une résine ayant pour matrice un gel de polymère de méthacrylate réticulé, sur lequel est greffée de la protéine A,
b) une étape d'inactivation virale,
c) une étape de chromatographie échangeuse de cations sur une résine ayant pour matrice un gel d'agarose réticulé, sur lequel sont greffés des groupements sulfonate (-SO₃-) par l'intermédiaire de bras espaceurs à base de dextrane,
d) une étape de chromatographie échangeuse d'anions sur une membrane hydrophile de polyéthersulfone revêtue d'un polymère réticulé sur lequel sont greffés des groupements amine quaternaire (Q), et
e) une étape de nanofiltration avec un filtre ayant une double membrane de polyéthersulfone d'une porosité d'environ 20 nm.

### Matériau de départ

Le procédé selon l'invention est applicable aussi bien à la purification d'un anticorps monoclonal que d'une protéine de fusion entre le fragment Fc d'un anticorps et un second polypeptide. En effet, la première étape (étape a)) est une étape de chromatographie d'affinité sur une résine porteuse de protéine A, une protéine de *Staphylococcus aureus* qui se lie spécifiquement au fragment Fc des anticorps, et en particulier au fragment Fc humain.

### Anticorps

Par « anticorps » ou « immunoglobuline », on entend une molécule comprenant au moins un domaine de liaison à un antigène donné et un domaine constant comprenant un fragment Fc capable de se lier aux récepteurs FcR. Chez la plupart des mammifères, comme l'homme et la souris, un anticorps est composé de 4 chaînes polypeptidiques : 2 chaînes lourdes et 2 chaînes légères reliées entre elles par un nombre variable de ponts disulfures assurant une flexibilité à la molécule. Chaque chaîne légère est constituée d'un domaine constant (CL) et d'un domaine variable (VL); les chaînes lourdes étant composées d'un domaine variable (VH) et de 3 ou 4 domaines constants (CH1 à CH3 ou CH1 à CH4) selon l'isotype de l'anticorps. Chez quelques rares mammifères, comme les chameaux et les lamas, les anticorps sont constitués de seulement deux chaines lourdes, chaque chaine lourde comprenant un domaine variable (VH) et une région constante.
Les domaines variables sont impliqués dans la reconnaissance de l'antigène, tandis que les domaines constants sont impliqués dans les propriétés biologiques, pharmacocinétiques et effectrices, de l'anticorps.
Contrairement aux domaines variables dont la séquence varie fortement d'un anticorps à un autre, les domaines constants sont caractérisés par une séquence en acides aminés très proche d'un anticorps à l'autre, caractéristique de l'espèce et de l'isotype, avec éventuellement quelques mutations somatiques. Le fragment Fc est naturellement composé de la région constante de la chaine lourde à l'exclusion du domaine CH1, c'est-à-dire de la région charnière inférieure et des domaines constants CH2 et CH3 ou CH2 à CH4 (selon l'isotype). Chez les IgG1 humaines, le fragment Fc complet est composé de la partie C-terminale de la chaine lourde à partir du résidu cystéine en position 226 (C226), la numérotation des résidus d'acides aminés dans le fragment Fc étant dans toute la présente description celle de l'index EU décrit dans Edelman et al (Edelman, G.M. et al., Proc. Natl. Acad. USA, 63, 78-85 (1969)) et Kabat et al (Kabat et al., Sequences of Proteins of Immunological Interest, 5th Ed. Public Health Service, National Institutes of Health, Bethesda, Md. (1991)). Les fragments Fc correspondants d'autres types d'immunoglobulines peuvent être identifiés aisément par l'homme du métier par des alignements de séquences.
Le fragment Fcγ est glycosylé au niveau du domaine CH2 avec la présence, sur chacune des 2 chaînes lourdes, d'un *N*-glycanne lié au résidu asparagine en position 297 (Asn 297).
Les domaines de liaison suivants, situés dans le Fcγ, sont importants pour les propriétés biologiques de l'anticorps :
- domaine de liaison au récepteur FcRn, impliqué dans les propriétés pharmacocinétiques (demi-vie in vivo) de l'anticorps :
   Différentes données suggèrent que certains résidus situés à l'interface des domaines CH2 et CH3 sont impliqués dans la liaison au récepteur FcRn.
- domaine de liaison à la protéine du complément C1q, impliqué dans la réponse CDC (pour « cytotoxicité dépendante du complément ») : situé dans le domaine CH2;
- domaine de liaison aux récepteurs FcR, impliqué dans les réponses de type phagocytose ou ADCC (pour « cytotoxicité cellulaire dépendante de l'anticorps) : situé dans le domaine CH2.
Au sens de l'invention, le fragment Fc d'un anticorps peut être naturel, tel que défini ci-dessus, ou bien avoir été modifié de diverses façons, à condition d'être capable de se lier à la protéine A. Les modifications peuvent inclure la délétion de certaines parties du fragment Fc, pourvu que le fragment Fc ainsi obtenu soit capable de se lier à la protéine A. Les modifications peuvent également inclure différentes substitutions d'acides aminés susceptibles d'affecter les propriétés biologiques de l'anticorps, pourvu que le fragment Fc ainsi obtenu soit capable de se lier à la protéine A. En particulier, lorsque l'anticorps est un IgG, il peut comprendre des mutations destinées à augmenter la liaison au récepteur FcγRIII (CD16), telles que décrites dans WO00/42072, Shields et al-2001, Lazar et al (Lazar, G. A., et al. Proc Natl Acad Sci U S A. 103(11): 4005-10), WO2004/029207, WO/2004063351, WO2004/074455. Des mutations permettant d'augmenter la liaison au récepteur FcRn et donc la demi-vie *in vivo* peuvent également être présentes, comme décrit par exemple dans Shields et al (Shields RL, et al. J Biol Chem. 2001 Mar 2;276(9):6591-604), Dall'Acqua et al-2002, Hinton et al-2004, Dall'Acqua et al-2006(a), WO00/42072, WO02/060919A2, WO2010/045193, ou WO2010/106180A2. D'autres mutations, comme celles permettant de diminuer ou d'augmenter la liaison aux protéines du complément et donc la réponse CDC, peuvent ou non être présentes (voir WO99/51642 ; WO2004074455A2 ; Idusogie EE et al. J Immunol. 2001; 166:2571-5 ; Dall'Acqua et al. J Immunol 2006; 177:1129-1138 ; et Moore GL. Et al. mAbs 2:2, 181-189; March/April, 2010).
Par « anticorps monoclonal » ou « composition d'anticorps monoclonal », on entend une composition comprenant des molécules d'anticorps possédant une spécificité antigénique identique et unique. Les molécules d'anticorps présentes dans la composition sont susceptibles de varier au niveau de leurs modifications post-traductionnelles, et notamment au niveau de leurs structures de glycosylation ou de leur point isoélectrique, mais ont toutes été codées par les mêmes séquences de chaines lourde et légère et ont donc, avant toute modification post-traductionnelle, la même séquence protéique. Certaines différences de séquences protéique, liées à des modifications post-traductionnelles (comme par exemple le clivage de la lysine C-terminale de la chaine lourde, la déamidation de résidus asparagine et/ou l'isomérisation de résidus aspartate), peuvent néanmoins exister entre les différentes molécules d'anticorps présentes dans la composition.
L'anticorps monoclonal purifié dans le cadre de l'invention peut avantageusement être chimérique, humanisé, ou humain. En effet, la protéine A de *Staphylococcus aureus* possède une affinité particulière de liaison aux fragments Fc humains, et en particulier au fragment Fcγ humain.
Par anticorps « chimérique », on entend désigner un anticorps qui contient une région variable (chaîne légère et chaîne lourde) naturelle dérivée d'un anticorps d'une espèce donnée en association avec les régions constantes de chaîne légère et chaîne lourde d'un anticorps d'une espèce hétérologue à ladite espèce donnée. Avantageusement, si la composition d'anticorps monoclonal pour son utilisation en tant que médicament selon l'invention comprend un anticorps monoclonal chimérique, celui-ci comprend des régions constantes humaines. Partant d'un anticorps non humain, un anticorps chimérique peut être préparé en utilisant les techniques de recombinaison génétique bien connues de l'homme du métier. Par exemple, l'anticorps chimérique pourra être réalisé en clonant pour la chaine lourde et la chaine légère un ADN recombinant comportant un promoteur et une séquence codant pour la région variable de l'anticorps non humain, et une séquence codant pour la région constante d'un anticorps humain. Pour les méthodes de préparation d'anticorps chimériques, on pourra par exemple se référer au document Verhoeyn et al (Verhoeyn et al. BioEssays, 8 : 74, 1988).
Par anticorps « humanisé », on entend désigner un anticorps qui contient des régions CDRs dérivées d'un anticorps d'origine non humaine, les autres parties de la molécule d'anticorps étant dérivées d'un (ou de plusieurs) anticorps humains. En outre, certains des résidus des segments du squelette (dénommés FR) peuvent être modifiés pour conserver l'affinité de liaison (Jones et al. Nature, 321 : 522-525, 1986; Verhoeyen et al-1988 ; Riechmann et al. Nature, 332 : 323-327, 1988). Les anticorps humanisés selon l'invention peuvent être préparés par des techniques connues de l'homme de l'art telles les technologies de « CDR grafting », de « resurfacing », de SuperHumanisation, de « Human string content », de « FR libraries », de « Guided selection », de « FR shuffling » et de « Humaneering », comme résumé dans la revue de Almagro et al (Almagro et al. Frontiers in Bioscience 13, 1619-1633, January 1, 2008).
Par anticorps « humain », on entend un anticorps dont toute la séquence est d'origine humaine, c'est-à-dire dont les séquences codantes ont été produites par recombinaison de gènes humains codant pour les anticorps. En effet, il est maintenant possible de produire des animaux transgéniques (par ex. des souris) qui sont capables, sur immunisation, de produire un répertoire complet d'anticorps humains en l'absence de production endogène d'immunoglobuline (voir Jakobovits et al., Proc. Natl. Acad. Sci. USA. 90:2551 (1993); Jakobovits et al., Nature, 362:255-258 (1993); Bruggermann et al., Year in Immuno., 7:33 (1993); Duchosal et al. Nature 355:258 (1992) ; brevets US5,591,669 ; US 5,598,369 ; US 5,545,806 ; US 5,545,807 ; US 6,150,584). Les anticorps humains peuvent aussi être obtenus à partir de banques de présentation de phages (Hoogenboom et al., J. Mol. Biol., 227:381 (1991); Marks et al., J. Mol. Biol., 222:581- 5 597 (1991); Vaughan et al. Nature Biotech 14:309 (1996)).
Les anticorps peuvent être plusieurs isotypes, en fonction de la nature de leur région constante : les régions constantes γ, α, µ, ε et δ correspondent respectivement aux immunoglobulines IgG, IgA, IgM, IgE et IgD. Avantageusement, l'anticorps monoclonal présent dans une composition utilisée en tant que médicament dans le cadre de l'invention est d'isotype IgG. En effet, cet isotype montre une capacité à engendrer une activité ADCC (« Antibody-Dependent Cellular Cytotoxicity », soit Cytotoxicité cellulaire dépendante de l'anticorps) chez le plus grand nombre d'individus (humains) et est donc principalement utilisé pour les applications pharmaceutiques d'anticorps monoclonaux. De plus, la protéine A possède une affinité particulière de liaison au fragment Fcγ humain.
Les régions constantes γ comprennent plusieurs sous-types : γ1, γ2, γ3, ces trois types de régions constantes présentant la particularité de fixer le complément humain, et γ4, créant ainsi les sous-isotypes IgG1, IgG2, IgG3, et IgG4. Avantageusement, l'anticorps monoclonal présent dans une composition utilisée en tant que médicament dans le cadre de l'invention est d'isotype IgG1. En effet, le fragment Fcγ1 possède une affinité de liaison particulièrement importante pour la protéine A.

La composition d'anticorps monoclonal à purifier par le procédé selon l'invention peut être produite par un clone cellulaire, un animal non-humain transgénique ou une plante transgénique, par des technologies bien connues de l'homme du métier.

Notamment, des clones cellulaires produisant la composition d'anticorps à purifier peuvent être obtenus par 3 technologies principales :
1) Obtention d'un hybridome par fusion d'un lymphocyte B produisant l'anticorps d'intérêt avec une lignée immortalisée,
2) Immortalisation d'un lymphocyte B produisant l'anticorps d'intérêt par le virus Epstein-Barr (EBV),
3) Isolement des séquences codant pour un anticorps d'intérêt (généralement à partir d'un hybridome ou d'un lymphocyte B immortalisé), clonage dans un ou plusieurs vecteur(s) d'expression des séquences codant pour les chaines lourde et légère de l'anticorps, transformation d'une lignée cellulaire par le(s) vecteur(s) d'expression, et séparation des différents clones cellulaires obtenus. Un vecteur d'expression des chaines lourde et légère de l'anticorps comprend les éléments nécessaires à l'expression des séquences codant pour les chaines lourde et légère de l'anticorps, et notamment un promoteur, un codon d'initiation de la transcription, des séquences de terminaison, et des séquences de régulation de la transcription appropriées. Ces éléments varient en fonction de l'hôte servant pour l'expression et sont choisis aisément par l'homme du métier au vu de ses connaissances générales. Le vecteur peut notamment être plasmidique ou viral. Les techniques de transformations sont également bien connues de l'homme du métier.
La transformation de lignées cellulaires par un ou plusieurs vecteur(s) d'expression des séquences codant pour les chaines lourde et légère de l'anticorps est la plus communément utilisée, en particulier pour l'obtention d'anticorps chimériques ou humanisés.
La lignée cellulaire transformée est de préférence d'origine eucaryote, et peut notamment être choisie parmi les cellules d'insecte, de plantes, de levure ou de mammifères. La composition d'anticorps peut alors être produite en cultivant la cellule hôte dans des conditions appropriées. Des lignées cellulaires appropriées pour la production d'anticorps incluent notamment les lignées choisies parmi : SP2/0 ; YB2/0 ; IR983F ; le myélome humain Namalwa ; PERC6 ; les lignées CHO, notamment CHO-K-1, CHO-Lec10, CHO-Lec1, CHO-Lec13, CHO Pro-5, CHO dhfr-, ou lignée CHO délétée pour les deux allèles codant pour le gène FUT8 et/ou le gène GMD ; Wil-2; Jurkat; Vero; Molt -4; COS-7; 293-HEK; BHK; K6H6; NSO; SP2/0-Ag 14, P3X63Ag8.653, lignée de cellule de canard embryonnaire EB66® (Vivalis) ; et lignées d'hépatome de rat H4-II-E (DSM ACC3129), H4-II-Es (DSM ACC3130) (voir WO2012/041768) Dans un mode de réalisation préféré, l'anticorps est produit dans l'une des lignées suivantes : YB2/0 ; lignée CHO délétée pour les deux allèles codant pour le gène FUT8 et/ou le gène GMD ; lignée de cellule de canard embryonnaire EB66® (Vivalis) ; et lignées d'hépatome de rat H4-II-E (DSM ACC3129), H4-II-Es (DSM ACC3130). Dans un mode de réalisation préféré, l'anticorps est produit dans YB2/0 (ATCC CRL-1662).

Alternativement, la composition d'anticorps à purifier peut être produite dans un animal non-humain transgénique.
Un animal non-humain transgénique peut être obtenu par injection directe du ou des gène(s) d'intérêt (ici, les gènes réarrangés codant pour les chaines lourde et légère de l'anticorps) dans un oeuf fertilisé (Gordon et al-1980). Un animal non-humain transgénique peut également être obtenu par introduction du ou des gène(s) d'intérêt (ici, les gènes réarrangés codant pour les chaines lourde et légère de l'anticorps) dans une cellule souche embryonnaire et préparation de l'animal par une méthode d'agrégation de chimère ou une méthode d'injection de chimère (voir Manipulating the Mouse Embryo, A Laboratory Manual, Second edition, Cold Spring Harbor Laboratory Press (1994); Gene Targeting, A Practical Approach, IRL Press at Oxford University Press (1993)). Un animal non-humain transgénique peut également être obtenu par une technique de clonage dans laquelle un noyau, dans lequel le ou les gène(s) d'intérêt (ici, les gènes réarrangés codant pour les chaines lourde et légère de l'anticorps) a été introduit, est transplanté dans un oeuf énucléé (Ryan et aL, 1997 Science; 278: 873 - 876; Cibelli et al., 1998 Science, 280 : 1256-1258 ; WO0026357A2). Un animal non humain transgénique produisant un anticorps d'intérêt peut être préparé par les méthodes ci-dessus. L'anticorps peut alors être accumulé dans l'animal transgénique et récolté, notamment à partir du lait ou des oeufs de l'animal. Pour la production d'anticorps dans le lait d'animaux non humains transgéniques, des procédés de préparation sont notamment décrits dans WO9004036A1, WO9517085A1, WO0126455A1, WO2004050847A2, WO2005033281A2, WO2007048077A2. Des procédés de purification de protéines d'intérêt à partir du lait sont également connus (voir WO0126455A1, WO2007106078A2). Les animaux non humains transgéniques d'intérêt incluent notamment la souris, le lapin, le rat, la chèvre, les bovins (notamment la vache), et les volailles (notamment le poulet).

La composition d'anticorps à purifier peut également être produite dans une plante transgénique. De nombreux anticorps ont déjà été produits dans des plantes transgéniques et les technologies nécessaires à l'obtention d'une plante transgénique exprimant un anticorps d'intérêt et à la récupération de l'anticorps sont bien connues de l'homme du métier (voir Stoger E, et al. Molecular Breeding 9: 149-158, 2002 ; Fisher R, et al. Vaccine 21 (2003) 820-825 ; Ma JK, et al. Nat Rev Genet. 2003 Oct;4(10):794-805 ; Schillberg S, et al. Vaccine 23 (2005) 1764-1769). Il est également possible d'influencer la glycosylation obtenue dans les plantes pour obtenir une glycosylation proche de celle des anticorps humains naturels (sans xylose), mais avec en outre une faible fucosylation, par exemple à l'aide de petits ARNs interférents (Forthal et al, J Immunol 2010;185;6876-6882).

L'anticorps monoclonal à purifier peut être dirigé contre tout antigène d'intérêt, et notamment contre les antigènes suivants :
- Rhésus D, les anticorps anti-Rhésus D étant utiles pour la prévention de l'alloimmunisation chez les individus Rhésus-négatifs,
- Antigènes exprimés par des cellules cancéreuses, susceptibles d'être ciblés dans le traitement de cancers, et notamment : CD20, Her2/neu, CD52, EGFR, EPCAM, CCR4, CTLA-4 (CD152), CD19, CD22, CD3, CD30, CD33, CD4, CD40, CD51 (Integrin alpha-V), CD80, CEA, FR-alpha, GD2, GD3, HLA-DR, IGF1R (CD221), phosphatidylserine, SLAMF7 (CD319), TRAIL-R1, TRAIL-R2.
- Antigènes exprimés par des cellules infectées par des agents pathogènes, susceptibles d'être ciblés dans le traitement d'infections par des agents pathogènes, et notamment : antigènes de *Clostridium difficile,* antigènes de *Staphylococcus aureus* (notamment ClfA et acide lipotheicoïque), antigènes du cytomégalovirus (notamment la glycoprotéine B), antigènes d'*Escherichia coli* (notamment toxine Shiga-like, sous unité IIB), antigènes du virus respiratoire syncytial (Protéine F notamment), antigènes du virus de l'hépatite B, antigènes du virus Influenza A (Hémagglutinine notamment), antigènes de *Pseudomonas aeruginosa* sérotype IATS O11, antigènes du virus de la rage (Glycoprotéine notamment), phosphatidylserine.
- Antigènes exprimés par des cellules immunitaires, susceptibles d'être ciblés pour le traitement de maladies autoimmunes, et notamment : CD20, CD52, CD25, CD2, CD22, CD3, et CD4.

### Protéine de fusion Fc

Par « protéine de fusion entre le fragment Fc d'un anticorps et un second polypeptide » ou « protéine de fusion Fc », on entend une protéine comprenant un fragment Fc d'anticorps lié de manière opérationnelle à un second polypeptide. Une telle protéine de fusion Fc comprend un fragment Fc lui conférant les propriétés effectrices et pharmacologiques d'un anticorps (ainsi que la capacité à se lier à la protéine A), et un second polypeptide (partenaire de fusion) lui conférant d'autres propriétés biologiques. Les protéines de fusion Fc comprennent, tout comme les anticorps monoclonaux, un fragment Fc se liant à la protéine A. Par conséquent, les enseignements techniques obtenus par les inventeurs sur des anticorps monoclonaux s'appliquent directement aux protéines de fusion Fc.

Le second polypeptide ou partenaire de fusion peut notamment être choisi parmi un récepteur (ou le domaine de liaison d'un récepteur à son ligand), un ligand (ou le domaine de liaison d'un ligand à son récepteur), une molécule d'adhésion, une cytokine, une chémokine, ou tout autre protéine ou domaine d'une protéine.

La fusion entre le fragment Fc et le second polypeptide peut être directe ou indirecte via un linker, qui peut notamment être constitué d'un ou plusieurs acides aminés de type glycine ou serine.

De telles protéines de fusion Fc ont été développées pour différentes applications thérapeutiques. Notamment, les protéines de fusion Fc suivantes sont susceptibles d'être purifiées à l'aide du procédé selon l'invention :
- abatacept et bélatacept: protéines de fusion entre l'ectodomaine de CTLA-4 et le Fc d'une IgG1, utilisées dans l'immunosuppresssion dans la polyarthrite rhumatoïde (abatacept) et transplantation (bélatacept)
- etanercept : protéine de fusion entre l'ectodomaine du récepteur TNF-RII et le Fc d'une IgG1, utlisé dans le traitement de la polyarthrite rhumatoïde et du psoriasis,
- alefacept : protéine de fusion entre l'ectodomaine de LFA-3 (CD58) et le Fc d'une IgG1, utilisé en thérapeutique dans le traitement du psoriasis, ou
- rilonacept : protéine de fusion dimérique constituée des domaines de liaison des parties extracellulaires du récepteur de type I de l'interleukine-1 (IL-1RI) et d'une protéine accessoire du récepteur de l'IL-1 (IL-1RAcP) liés linéairement à la portion Fc de l'immunoglobuline humaine IgG1, utilisée dans le traitement des formes sévères des syndromes périodiques associés à la cryopyrine (CAPS), incluant le syndrome familial auto-inflammatoire au froid (FCAS) et le syndrome de Muckle-Wells (MWS).
- atacicept : de fusion recombinante qui contient le récepteur soluble TACI associé au fragment Fc d'une IgG1 humaine, utilisé dans le traitement de la polyarthrite rhumatoïde, le lupus systémique et la sclérose en plaques.
- briobacept : protéine de fusion constituée du récepteur BAFF et d'un fragment constant d'IgG1, utilisé dans le traitement de la polyarthrite rhumatoïde.

De telles protéines de fusion Fc sont produites de façon recombinante, par toute technologie appropriée choisie notamment parmi celles décrites ci-dessus pour la production d'anticorps monoclonaux recombinants (clone cellulaire transformé, animal non-humain transgénique, plante transgénique notamment).

### Produit à purifier

Le procédé de purification selon l'invention est avantageusement mis en oeuvre à partir d'une composition comprenant l'anticorps sous forme non purifiée, c'est à dire comprenant en outre d'autres produits contaminants (autres protéines, ADN, sucres, etc...).

Le procédé selon l'invention peut ainsi notamment être mis en oeuvre à partir des matériaux suivants :
- surnageant de culture d'un clone producteur de l'anticorps monoclonal ou de la protéine de fusion entre le fragment Fc d'un anticorps et un second polypeptide,
- lait d'un animal non-humain transgénique exprimant l'anticorps monoclonal ou la protéine de fusion entre le fragment Fc d'un anticorps et un second polypeptide, ou
- extrait cellulaire d'une plante transgénique exprimant l'anticorps monoclonal ou la protéine de fusion entre le fragment Fc d'un anticorps et un second polypeptide.

De nombreux anticorps monoclonaux ou protéines de fusion entre le fragment Fc d'un anticorps et un second polypeptide sont produits dans des clones cellulaires transformés, et le procédé selon l'invention peut donc avantageusement être mis en oeuvre sur un surnageant de culture d'un clone producteur de l'anticorps monoclonal ou de la protéine de fusion entre le fragment Fc d'un anticorps et un second polypeptide. Par « clone producteur de l'anticorps monoclonal ou de la protéine de fusion entre le fragment Fc d'un anticorps et un second polypeptide » on entend une cellule transformée (qui peut notamment être choisie parmi celles décrites ci-dessus) par un vecteur d'expression de l'anticorps monoclonal ou de la protéine de fusion entre le fragment Fc d'un anticorps et un second polypeptide. Par « surnageant de culture », on entend la composition obtenue par centrifugation du milieu de culture des cellules du clone producteur et exclusion des cellules ou débris présents dans le milieu de culture, ledit milieu de culture pouvant optionnellement avoir été soumis préalablement à une étape de lyse des cellules du clone producteur.

### Etape a)

L'étape a) du procédé selon l'invention est une étape de chromatographie d'affinité sur une résine ayant pour matrice un gel de polymère de méthacrylate réticulé, sur lequel est greffée de la protéine A. Cette étape permet de purifier de façon très importante l'anticorps ou la protéine de fusion Fc, du fait de la forte affinité et de la forte spécificité de la protéine A pour le fragment Fc des anticorps.

### Protéine A

La chromatographie d'affinité permet de séparer spécifiquement les molécules se liant à un ligand particulier. Dans le cadre de la purification d'anticorps, une étape de chromatographie d'affinité à la protéine A est couramment utilisée, cette protéine se liant spécifiquement au fragment Fc des anticorps, en particulier au fragment Fc humain, et plus particulièrement au fragment Fcγ et notamment Fcγ1 humain.

Par « protéine A », on entend la protéine de *Staphylococcus aureus* codée par le gène *Spa*, ou un dérivé ou un fragment de cette protéine capable de se lier au fragment Fc d'un anticorps monoclonal. La protéine A de *Staphylococcus aureus* codée par le gène *spa* est une protéine membranaire de *Staphylococcus aureus* comprenant en N-terminal 5 domaines homologues (E-D-A-B-C) capables chacun de se lier au fragment Fc des anticorps, la région C-terminale (X) servant à ancrer la protéine dans la membrane bactérienne. Une protéine A native peut être isolée directement de cultures de *Staphylococcus aureus* sécrétant de la protéine A, ou de culture de bactéries *Escherichia coli* (*E. coli*) recombinantes exprimant la protéine A (Löfdahl et al. Proc Natl Acad Sci U S A. 1983 Feb;80(3):697-701 ; Uhlén et al. J Biol Chem. 1984 Feb 10;259(3):1695-702). Afin d'optimiser son utilisation pour la chromatographie d'affinité, différents fragments ou variants de protéines A capables de se lier spécifiquement et avec une forte affinité au fragment Fc des anticorps ont été proposés. En particulier, différents domaines de la protéine A native ou dérivés et/ou fragments de ces domaines ont été proposés sous forme répétée (dimères, tétramères ou héxamères notamment) pour la purification d'anticorps. Ainsi, un fragment « Z » du domaine B de la protéine A a été proposé pour être utilisé dans la purification d'anticorps (US6,013,763). Différents variants de protéine A recombinante ou de fragment de protéine a recombinante comprenant un résidu cystéine ou un résidu arginine en N terminal permettant un attachement plus facile à la matrice de chromatographie ont également été décrits (US5,084,559 ; US5,260,373 ; US6,399,750). Des variants de protéine A ou de fragments fonctionnels de protéine A ayant une stabilité améliorée en conditions alkalines ont également décrits (US7,709,209 ; WO2012/083425), ces variants étant utiles pour permettre une sanitisation répétée des colonnes de chromatographie d'affinité à la protéine A sans relargage trop important de protéine A.

### Matrice de chromatographie d'affinité

Dans le procédé selon l'invention, la protéine A est attachée à une matrice de chromatographie d'affinité constituée d'un gel de polymère de méthacrylate réticulé.

Par « polymère de méthacrylate réticulé », on entend tout polymère ou co-polymère réticulé comprenant des monomères de méthacrylate. Par « méthacrylate », on entend l'ion méthacrylate de formule (CH₂=C(CH₃)COO⁻), ainsi que les sels et les esters de cet ion.

En effet, les inventeurs ont mis en évidence que ce type particulier de résine permet d'augmenter la charge en anticorps purifié en une seule fois, de garantir un bon rendement (an moins 90%) et d'obtenir un éluat à l'aspect limpide (voir Exemple 1).

Le gel de polymère de méthacrylate réticulé sur lequel est greffée de la protéine A utilisé à l'étape a) peut avantageusement se présenter sous forme de billes ayant un diamètre moyen compris entre 30 et 60 µm, avantageusement entre 40 et 50 µm, et notamment d'environ 45, 49 ou 50 µm.

Selon la protéine A utilisée, celle-ci peut être attachée à la matrice de chromatographie d'affinité par différents types de couplage usuels, comme le couplage multipoint par CNBr (couplage entre des fonctions amino primaires de la protéine A et une matrice activée par CNBr), le couplage à point unique par une liaison thioéther entre la matrice et un résidu cystéine de la protéine A, obtenu notamment par activation de la matrice par un epoxide ou par une épichlorohydrine.

Des exemples de matrices constituées d'un gel de polymère de méthacrylate réticulé sous forme de billes ayant un diamètre moyen compris entre 40 et 50 µm, sur lequel est greffée de la protéine A, incluent les matrices suivantes : TOYOPEARL® AF-rProtein A HC-650F (matrice de polymère de méthacrylate hydroxylé sous forme de billes d'un diamètre moyen de 45 µm, sur laquelle est greffée de la protéine A recombinante, commercialisée par TOSOH BIOSCIENCE), Amsphere™ Protein A JWT203 (résine de polymère de méthacrylate sous forme de billes d'un diamètre moyen de 49 µm, sur laquelle est greffée un tétramère de domaine C modifié stable en conditions alkalines produit dans *E. coli,* commercialisée par JSR Corporation), Amsphere™ Protein A A3 (résine de copolymère de méthacrylate et d'un monomère hydrophile sous forme de billes d'un diamètre moyen compris entre 20 et 80 µm, de préférence entre 30 et 70 µm, entre 40 et 60 µm, ou entre 40 et 50 µm, sur laquelle est greffée un tétramère de domaine C modifié stable en conditions alkalines produit dans *E. coli,* commercialisée par JSR Corporation). Avantageusement, la résine ayant pour matrice un gel de polymère de méthacrylate réticulé, sur lequel est greffée de la protéine A est choisie parmi :
- une matrice de polymère de méthacrylate hydroxylé sous forme de billes d'un diamètre moyen de 45 µm, sur laquelle est greffée de la protéine A recombinante (notamment la résine TOYOPEARL® AF-rProtein A HC-650F) ;
- une résine de polymère de méthacrylate sous forme de billes d'un diamètre moyen de 49 µm, sur laquelle est greffée un tétramère de domaine C modifié stable en conditions alkalines produit dans *E. coli* (notamment la résine Amsphere™ Protein A JWT203), et
- une résine de copolymère de méthacrylate et d'un monomère hydrophile sous forme de billes d'un diamètre moyen compris entre 20 et 80 µm, de préférence entre 30 et 70 µm, entre 40 et 60 µm, ou entre 40 et 50 µm, sur laquelle est greffée un tétramère de domaine C modifié stable en conditions alkalines produit dans *E. coli* (notamment la résine Amsphere™ Protein A A3).

Plus avantageusement, la résine est :
- une résine de polymère de méthacrylate sous forme de billes d'un diamètre moyen de 49 µm, sur laquelle est greffée un tétramère de domaine C modifié stable en conditions alkalines produit dans *E*. coli (notamment la résine Amsphere™ Protein A JWT203), ou
- résine de copolymère de méthacrylate et d'un monomère hydrophile sous forme de billes d'un diamètre moyen compris entre 20 et 80 µm, de préférence entre 30 et 70 µm, entre 40 et 60 µm, ou entre 40 et 50 µm, sur laquelle est greffée un tétramère de domaine C modifié stable en conditions alkalines produit dans *E. coli* (notamment la résine Amsphere™ Protein A A3).

Encore plus avantageusement, la résine est une résine de copolymère de méthacrylate et d'un monomère hydrophile sous forme de billes d'un diamètre moyen compris entre 20 et 80 µm, de préférence entre 30 et 70 µm, entre 40 et 60 µm, ou entre 40 et 50 µm, sur laquelle est greffée un tétramère de domaine C modifié stable en conditions alkalines produit dans *E. coli* (notamment la résine Amsphere™ Protein A A3). Une telle résine possède en effet une forte capacité de charge et permet d'obtenir des éluats avec un bon rendement, une faible turbidité, et une bonne élimination des impuretés et notamment des autres protéines produites par la cellule de production (appelées « HCP » pour « Host cell proteins »), et de l'ADN de la cellule de production (appelé « HC-DNA » pour « Host cell DNA ») (voir Exemple 4).

### Lavage (optionnel)

L'étape a) du procédé selon l'invention peut en outre comprendre, de façon optionnelle mais préférée, une sous-étape de lavage de la résine ayant pour matrice un gel de polymère de méthacrylate réticulé, sur lequel est greffée de la protéine A, sur laquelle l'anticorps monoclonal ou la protéine de fusion entre le fragment Fc d'un anticorps et un second polypeptide s'est fixé.

Un tel lavage permet notamment d'améliorer l'élimination des impuretés, et notamment des autres protéines produites par la cellule de production (appelées « HCP » pour « Host cell proteins »), et de l'ADN de la cellule de production (appelé « HC-DNA » pour « Host cell DNA »).

Ce lavage est avantageusement réalisé à l'aide d'une solution saline comprenant une concentration en NaCl d'au moins 1M, avantageusement au moins 1,2M, au moins 1,3M, au moins 1,4M, au moins 1,5M, au moins 1,6M, voire au moins 1,7M. Avantageusement, la concentration en NaCl de la solution saline utilisée pour le lavage est comprise entre 1M et 2,5M, entre 1M et 2,1M, entre 1M et 2M, entre 1M et 1,9M, entre 1M et 1,8M, entre 1M et 1,7M, entre 1,1M et 2,5M, entre 1,1M et 2,1M, entre 1,1M et 2M, entre 1,1M et 1,9M, entre 1,1M et 1,8M, entre 1,1M et 1,7M, entre 1,2M et 2,5M, entre 1,2M et 2,1M, entre 1,2M et 2M, entre 1,2M et 1,9M, entre 1,2M et 1,8M, entre 1,2M et 1,7M, entre 1,3M et 2,5M, entre 1,3M et 2M, entre 1,3M et 1,9M, entre 1,3M et 1,8M, entre 1,3M et 1,7M, entre 1,4M et 2,5M, entre 1,4M et 2M, entre 1,4M et 1,9M, entre 1,4M et 1,8M, entre 1,4M et 1,7M, entre 1,5M et 2,5M, entre 1,5M et 2M, entre 1,5M et 1,9M, entre 1,5M et 1,8M, entre 1,5M et 1,7M, entre 1,6M et 2,5M, entre 1,6M et 2M, entre 1,6M et 1,9M, entre 1,6M et 1,8M, entre 1,6M et 1,7M, entre 1,7M et 2,5M, entre 1,7M et 2M, entre 1,7M et 1,9M, entre 1,7M et 1,8M, et notamment d'environ 1,7M. On pourra notamment utiliser une solution ayant la composition suivante : Tampon Tris 25 mM, EDTA 5 mM, pH 7,1, et NaCl dans l'une des gammes de concentrations décrites ci-dessus.

Bien qu'un tel lavage puisse être utilisé pour toute matrice de chromatographie d'affinité constituée d'un gel de polymère de méthacrylate réticulé, il est particulièrement avantageux lorsque la matrice de chromatographie d'affinité est une résine de copolymère de méthacrylate et d'un monomère hydrophile sous forme de billes d'un diamètre moyen compris entre 20 et 80 µm, de préférence entre 30 et 70 µm, entre 40 et 60 µm, ou entre 40 et 50 µm, sur laquelle est greffée un tétramère de domaine C modifié stable en conditions alkalines produit dans *E. coli,* telle que la résine Amsphere™ Protein A A3 commercialisée par JSR Corporation.

### Tampon d'élution

Lors de l'étape a), la composition d'anticorps monoclonal ou de protéine de fusion Fc à purifier est injectée sur une résine ayant pour matrice un gel de polymère de méthacrylate réticulé, sur lequel est greffée de la protéine A, équilibrée par un tampon à pH neutre, à laquelle l'anticorps monoclonal ou la protéine de fusion Fc va se lier par l'intermédiaire du fragment Fc.
La résine est ensuite lavée pour éliminer les contaminants non liés à la résine, puis l'anticorps monoclonal ou la protéine de fusion Fc est élué à l'aide d'un tampon permettant de rompre la liaison entre la protéine A et le fragment Fc de l'anticorps ou de la protéine de fusion Fc.
Dans la présente description, différents types de tampons peuvent être utilisés pour l'élution. Notamment, l'élution peut être obtenue à pH acide, et des tampons utilisant différents acides faibles peuvent donc être utilisés pour l'élution.
Cependant, dans le cadre de la présente invention, un tampon formiate de sodium est utilisé. Dans le cadre de l'invention, le tampon formiate de sodium est utilisé à une molarité de 5 à 10 mM, avantageusement de 5 à 9 mM, de 5 à 8 mM, de 5 à 7 mM, de 5 à 6 mM, de 6 à 10 mM, de 6 à 9 mM, de 6 à 8 mM, de 6 à 7 mM, de 7 à 10 mM, de 7 à 9 mM, de 7 à 8 mM, de 8 à 10 mM, de 8 à 9 mM, ou de 9 à 10 mM, plus avantageusement de 5 à 9 mM, de 5 à 8 mM, de 5 à 7 mM, de 5 à 6 mM et notamment d'environ 5 mM. Dans le cadre de l'invention, le tampon formiate de sodium est utilisé à un pH compris entre 2,6 et 3,6, entre 2,7 et 3,6, entre 2,8 et 3,6, entre 2,9 et 3,6, entre 3,0 et 3,6, entre 3,1 et 3,6, entre 3,2 et 3,6, entre 3,3 et 3,6, entre 3,4 et 3,6, entre 3,5 et 3,6, entre 2,6 et 3,5, entre 2,7 et 3,5, entre 2,8 et 3,5, entre 2,9 et 3,5, entre 3,0 et 3,5, entre 3,1 et 3,5, entre 3,2 et 3,5, entre 3,3 et 3,5, entre 3,4 et 3,5, entre 2,6 et 3,4, entre 2,7 et 3,4, entre 2,8 et 3,4, entre 2,9 et 3,4, entre 3,0 et 3,4, entre 3,1 et 3,4, entre 3,2 et 3,4, entre 3,3 et 3,4, entre 2,6 et 3,3, entre 2,7 et 3,3, entre 2,8 et 3,3, entre 2,9 et 3,3, entre 3,0 et 3,3, entre 3,1 et 3,3, entre 3,2 et 3,3, entre 2,6 et 3,2, entre 2,7 et 3,2, entre 2,8 et 3,2, entre 2,9 et 3,2, entre 3,0 et 3,2, entre 3,1 et 3,2, entre 2,6 et 3,1, entre 2,7 et 3,1, entre 2,8 et 3,1, entre 2,9 et 3,1, entre 3,0 et 3,1, entre 2,6 et 3,0, entre 2,7 et 3,0, entre 2,8 et 3,0, entre 2,9 et 3,0, entre 2,6 et 2,9, entre 2,7 et 2,9, entre 2,8 et 2,9, entre 2,6 et 2,8, entre 2,7 et 2,8, ou entre 2,6 et 2,7, plus avantageusement entre 2,7 et 3,5, entre 2,8 et 3,4, entre 2,9 et 3,3, entre 3,0 et 3,2, voire d'environ 3,1, ou entre 3,6 et 4, ou encore entre 3,1 et 3,6.
Lorsque la matrice de chromatographie d'affinité est une résine de polymère de méthacrylate sous forme de billes d'un diamètre moyen de 49 µm, sur laquelle est greffée un tétramère de domaine C modifié stable en conditions alkalines produit dans *E. coli,* telle que la résine Amsphere™ Protein A JWT203 commercialisée par JSR Corporation, le pH optimal d'élution a été déterminé à environ 3,1, et toute gamme de pH d'élution mentionnée ci-dessus encadrant cette valeur optimale est préférée. Lorsque la matrice de chromatographie d'affinité est une résine de copolymère de méthacrylate et d'un monomère hydrophile sous forme de billes d'un diamètre moyen compris entre 20 et 80 µm, de préférence entre 30 et 70 µm, entre 40 et 60 µm, ou entre 40 et 50 µm, sur laquelle est greffée un tétramère de domaine C modifié stable en conditions alkalines produit dans *E. coli,* telle que la résine Amsphere™ Protein A A3 commercialisée par JSR Corporation, le pH optimal d'élution a été déterminé à environ 3,6, et toute gamme de pH d'élution mentionnée ci-dessus encadrant cette valeur optimale est préférée.
Dans le cadre de l'invention, le tampon formiate de sodium est utilisé :
- à une molarité de 5 à 10 mM, avantageusement de 5 à 9 mM, de 5 à 8 mM, de 5 à 7 mM, de 5 à 6 mM, de 6 à 10 mM, de 6 à 9 mM, de 6 à 8 mM, de 6 à 7 mM, de 7 à 10 mM, de 7 à 9 mM, de 7 à 8 mM, de 8 à 10 mM, de 8 à 9 mM, ou de 9 à 10 mM, plus avantageusement de 5 à 9 mM, de 5 à 8 mM, de 5 à 7 mM, de 5 à 6 mM et notamment d'environ 5 mM, et
- à un pH compris entre 2,6 et 3,6, entre 2,7 et 3,6, entre 2,8 et 3,6, entre 2,9 et 3,6, entre 3,0 et 3,6, entre 3,1 et 3,6, entre 3,2 et 3,6, entre 3,3 et 3,6, entre 3,4 et 3,6, entre 3,5 et 3,6, entre 2,6 et 3,5, entre 2,7 et 3,5, entre 2,8 et 3,5, entre 2,9 et 3,5, entre 3,0 et 3,5, entre 3,1 et 3,5, entre 3,2 et 3,5, entre 3,3 et 3,5, entre 3,4 et 3,5, entre 2,6 et 3,4, entre 2,7 et 3,4, entre 2,8 et 3,4, entre 2,9 et 3,4, entre 3,0 et 3,4, entre 3,1 et 3,4, entre 3,2 et 3,4, entre 3,3 et 3,4, entre 2,6 et 3,3, entre 2,7 et 3,3, entre 2,8 et 3,3, entre 2,9 et 3,3, entre 3,0 et 3,3, entre 3,1 et 3,3, entre 3,2 et 3,3, entre 2,6 et 3,2, entre 2,7 et 3,2, entre 2,8 et 3,2, entre 2,9 et 3,2, entre 3,0 et 3,2, entre 3,1 et 3,2, entre 2,6 et 3,1, entre 2,7 et 3,1, entre 2,8 et 3,1, entre 2,9 et 3,1, entre 3,0 et 3,1, entre 2,6 et 3,0, entre 2,7 et 3,0, entre 2,8 et 3,0, entre 2,9 et 3,0, entre 2,6 et 2,9, entre 2,7 et 2,9, entre 2,8 et 2,9, entre 2,6 et 2,8, entre 2,7 et 2,8, ou entre 2,6 et 2,7, plus avantageusement entre 2,7 et 3,5, entre 2,8 et 3,4, entre 2,9 et 3,3, entre 3,0 et 3,2, voire d'environ 3,1, ou entre 3,6 et 4, ou encore entre 3,1 et 3,6.
En effet, ce type de tampon permet d'obtenir un rendement satisfaisant, un éluat limpide ou faiblement opalescent et d'un volume satisfaisant, ainsi qu'une très faible proportion d'agrégats d'anticorps. Ce n'est pas le cas de tampons comme les tampons acétate de sodium dihydraté ou citrate trisodique dihydraté, qui conduisent à des éluats moyennement ou fortement opalescents, ou le tampon acide maléique, NaOH 0,5M qui conduit à une formation significative d'agrégats d'anticorps (voir Exemple 1).
De plus, ce type de tampon permet une bonne élimination des impuretés, et notamment des autres protéines produites par la cellule de production (appelées « HCP » pour « Host cell proteins »), et de l'ADN de la cellule de production (appelé « HC-DNA » pour « Host cell DNA ») (voir Exemple 4).
A l'issue de l'élution de l'anticorps ou de la protéine de fusion Fc, l'éluat peut être neutralisé, c'est-à-dire amené à un pH compris entre 5 et 7, notamment entre 5,5 et 6,5, et en particulier d'environ 6,0. Cette neutralisation peut notamment être réalisée en ajoutant une quantité appropriée de tampon Tris 1M à pH 7,5 ou de soude (NaOH) 1M.

Ainsi, les choix spécifiques réalisés par les inventeurs concernant la matrice de chromatographie d'affinité et le tampon d'élution permettent de diminuer significativement le coût de cette étape (charge augmentée, très bon rendement, matrice relativement bon marché), tout en garantissant une forte purification et une bonne qualité de l'anticorps purifié (éluat limpide ou faiblement opalescent, très faible proportion d'agrégats).

### Etape b)

L'étape b) du procédé selon l'invention est une étape d'inactivation virale.

Par « étape d'inactivation virale », on entend une étape dans laquelle les virus ne sont pas éliminés de la solution (des antigènes peuvent encore être détectés), mais sont rendus inactifs et donc inoffensifs. Ces étapes incluent notamment le chauffage à sec, la pasteurisation, et le traitement solvant-détergent ou par un détergent seul. Ces différentes étapes d'inactivation virale sont bien connues de l'homme du métier (voir notamment les directives de l'OMS concernant les procédures d'inactivation et d'élimination virale destinées à assurer la sécurité virale des produis dérivés du plasma sanguin humain, disponibles sur le site internet de l'OMS).

Avantageusement, dans le procédé selon l'invention, l'étape d'inactivation virale est une étape de traitement solvant-détergent ou de traitement par un détergent seul. Un traitement solvant-détergent est réalisée par traitement de la solution par un mélange de solvant, notamment le tri-(N-butyl)-phosphate (TnBP), et d'un détergent, notamment le Polysorbate 80 (Polyoxyéthylène (20) sorbitan monooléate) ou le polyoxyéthylène-p-t-octylphénol (Triton X-100, N° CAS 9002-93-1), dans des conditions appropriées. UN exemple d'étape de traitement solvant-détergent est réalisé en présence de 1% (poids/volume) de Polysorbate 80 et 0,3% (volume/volume) de TnBP pendant au moins 7 heures à 25±1 °C. L'étape d'inactivation virale peut également être réalisée par traitement par un détergent seul, tel que le Polysorbate 80 (Polyoxyéthylène (20) sorbitan monooléate) ou le polyoxyéthylène-p-t-octylphénol (Triton X-100, N° CAS 9002-93-1). Un exemple d'un tel traitement est une incubation pendant 30 à 120 minutes (en particulier pendant environ 1 heure) à une température de 20 à 25°C (notamment à une température d'environ 21 ou 22°C) dans un milieu comprenant 0,5 à 2% (v/v) (notamment environ 1% v/v) de polyoxyéthylène-p-t-octylphénol (Triton X-100, N° CAS 9002-93-1).

### Etape c)

L'étape c) vise à améliorer la purification de l'anticorps monoclonal ou de la protéine de fusion Fc en éliminant différents contaminants, tels que les protéines ou les acides nucléiques résiduels de l'hôte de production, la protéine A susceptible d'avoir été relarguée lors de l'étape a) ou le solvant et/ou le détergent susceptible d'avoir été utilisé à l'étape b).

L'étape c) du procédé selon l'invention est une étape de chromatographie échangeuse de cations sur une résine ayant pour matrice un gel d'agarose réticulé, sur lequel sont greffés des groupements sulfonate (-SO₃-) par l'intermédiaire de bras espaceurs à base de dextrane.

En effet, les inventeurs ont mis en évidence que l'utilisation d'une telle résine permet d'augmenter la charge d'anticorps monoclonal ou de protéine de fusion Fc susceptible d'être traitée en une seule fois, réduisant ainsi les coûts de purification (voir Exemple 2).

Dans cette étape, la composition d'anticorps monoclonal ou de protéine de fusion Fc issue de l'étape b) d'inactivation virale est appliquée sur une résine ayant pour matrice un gel d'agarose réticulé, sur lequel sont greffés des groupements sulfonate (-SO₃-) par l'intermédiaire de bras espaceurs à base de dextrane.

La conductivité et/ou le pH de la composition issue de l'étape b) d'inactivation virale peut avantageusement être ajustée avant application sur la résine. En particulier, la conductivité peut être ajustée à une valeur comprise entre 3 et 7 mS/cm, notamment entre 4 et 6 mS/cm et en particulier d'environ 5 mS/cm. L'ajustement de la conductivité peut notamment être réalisé en ajoutant une quantité appropriée d'eau purifiée, d'un tampon acétate de sodium ou avantageusement d'un tampon formiate. Le pH peut quant à lui être ajusté à une valeur comprise entre 5 et 7, notamment entre 5,5 et 6,5, et en particulier d'environ 6,0. L'ajustement du pH peut notamment être réalisé en ajoutant une quantité appropriée de soude (NaOH) 0,5M.

Le gel d'agarose réticulé, sur lequel sont greffés des groupements sulfonate (-SO₃-) par l'intermédiaire de bras espaceurs à base de dextrane utilisé à l'étape c) peut avantageusement se présenter sous forme de billes ayant un diamètre moyen compris entre 10 et 200 µm, avantageusement entre 50 et 150 µm, et notamment d'environ 90 µm.

Des exemples de matrices constituées d'un gel d'agarose réticulé, sur lequel sont greffés des groupements sulfonate (-SO₃-) par l'intermédiaire de bras espaceurs incluent les matrices suivantes : Capto™ S (matrice de gel d'agarose réticulé, sur lequel sont greffés des groupements sulfonate (-SO₃-) par l'intermédiaire de bras espaceurs à base de dextrane sous forme de billes d'un diamètre moyen de 90 µm, commercialisée par GE Healthcare Life Sciences), Fractogel® EMD SO₃⁻ (matrice de polymère de méthacrylate, sur lequel sont greffés des groupements sulfonate (-SO₃-) par l'intermédiaire de longues chaines de polymère linéaire d'acrylamide comprenant 15 à 50 unités d'acrylamide, sous forme de billes d'un diamètre moyen de 30 (type S) ou 65 (type M) µm), et Eshmuno®S (matrice de polyvinyléther réticulé hydrophile, sur laquelle sont greffés des groupements sulfonate (-SO₃-) par l'intermédiaire de bras espaceurs, sous forme de billes d'un diamètre moyen de 75-95 µm). Avantageusement, la résine ayant pour matrice un gel d'agarose réticulé, sur lequel sont greffés est choisie parmi une matrice de gel d'agarose réticulé, sur lequel sont greffés des groupements sulfonate (-SO₃-) par l'intermédiaire de bras espaceurs à base de dextrane sous forme de billes d'un diamètre moyen de 90 µm (résine Capto™ S notamment), une matrice de polymère de méthacrylate, sur lequel sont greffés des groupements sulfonate (-SO₃-) par l'intermédiaire de longues chaines de polymère linéaire d'acrylamide comprenant 15 à 50 unités d'acrylamide, sous forme de billes d'un diamètre moyen de 30 (type S) ou 65 (type M) µm (résine Fractogel® EMD SO₃⁻ notamment) et une matrice de polyvinyléther réticulé hydrophile, sur laquelle sont greffés des groupements sulfonate (-SO₃-) par l'intermédiaire de bras espaceurs, sous forme de billes d'un diamètre moyen de 75-95 µm (résine Eshmuno®S notamment), plus avantageusement la résine est une matrice de gel d'agarose réticulé, sur lequel sont greffés des groupements sulfonate (-SO₃-) par l'intermédiaire de bras espaceurs à base de dextrane sous forme de billes d'un diamètre moyen de 90 µm (résine Capto™ S notamment).

L'élution peut notamment être réalisée en augmentant la conductivité et/ou le pH. En particulier, le tampon d'élution peut posséder une conductivité comprise entre 16 à 20 mS/cm, notamment entre 17 et 19 mS/cm et en particulier d'environ 18 mS/cm. Le tampon d'élution peut posséder un pH compris entre 6 et 8, notamment entre 6,5 et 7,5, et en particulier d'environ 7,0. Il peut notamment s'agir d'un tampon Tris 20 mM, NaCl qsp conductivité 18 mS/cm et pH 7,0.

Le débit de l'étape de chromatographie est avantageusement ajusté à une valeur correspondant à un temps de résidence compris entre 1 et 3 minutes, avantageusement entre 1,5 et 2,5 minutes et notamment d'environ 2 minutes. En fonction du volume de gel, le débit approprié peut être calculé sur la base de la formule suivante : débit (mL/min) = volume de gel (mL)/ temps de résidence (min).

### Etape d)

L'étape d) du procédé selon l'invention vise à améliorer encore la purification de l'anticorps monoclonal ou de la protéine de fusion Fc en éliminant différents contaminants, tels que les protéines ou les acides nucléiques résiduels de l'hôte de production, la protéine A susceptible d'avoir été relarguée lors de l'étape a) ou le solvant et/ou le détergent susceptible d'avoir été utilisé à l'étape b). Elle est particulièrement efficace pour éliminer es acides nucléiques résiduels.

Il s'agit d'une étape de chromatographie échangeuse d'anions sur une membrane hydrophile de polyéthersulfone revêtue d'un polymère réticulé sur lequel sont greffés des groupements amine quaternaire (Q).

La membrane possède avantageusement une taille moyenne de pores comprise entre 0,5 et 1 µm, avantageusement entre 0,6 et 0,9 µm, entre 0,7 et 0,9 µm, et notamment d'environ 0,8 µm.

La membrane comprend avantageusement plusieurs couches de polyéthersulfone revêtue d'un polymère réticulé sur lequel sont greffés des groupements amine quaternaire (Q), avantageusement entre 10 et 20 couches, notamment entre 14 et 18 couches, et en particulier 16 couches.

Un exemple d'une telle membrane est la membrane Mustang ® Q (membrane hydrophile de 16 couches de polyéthersulfone ayant une taille moyenne de pores de 0,8 µm, revêtue d'un polymère réticulé sur lequel sont greffés des groupements amine quaternaire (Q)) commercialisée par Pall.

Dans cette étape d), la composition d'anticorps monoclonal ou de protéine de fusion Fc issue de l'étape c) de chromatographie échangeuse de cations est appliquée sur une membrane hydrophile de polyéthersulfone revêtue d'un polymère réticulé sur lequel sont greffés des groupements amine quaternaire (Q).

La conductivité et/ou le pH de la composition issue de l'étape c) de chromatographie échangeuse de cations peut avantageusement être ajustée avant application sur la membrane. En particulier, la conductivité peut être ajustée à une valeur comprise entre 8 et 12 mS/cm, notamment entre 9 et 11 mS/cm et en particulier d'environ 10 mS/cm. L'ajustement de la conductivité peut notamment être réalisé en ajoutant une quantité appropriée d'un tampon phosphate 20 mM ou avantageusement d'un tampon Tris 20 mM. Le pH peut quant à lui être ajusté à une valeur comprise entre 6 et 10, notamment entre 7,0 et 9,0, entre 7,5 et 8,5, et en particulier d'environ 8,0. L'ajustement du pH peut notamment être réalisé en ajoutant une quantité appropriée de soude (NaOH) 0,5M.

La membrane est avantageusement équilibrée par un tampon Tris, en particulier un tampon Tris ayant les caractéristiques suivantes :
- une concentration comprise entre 15 et 25 mM, entre 16 et 24 mM, entre 17 et 23 mM, entre 18 et 22 mM, entre 19 et 21 mM, en particulier d'environ 20 mM,
- un pH compris entre 6 et 10, entre 7,0 et 9,0, entre 7,5 et 8,5, en particulier d'environ 8,0,
- une conductivité comprise entre 8 et 12 mS/cm, notamment entre 9 et 11 mS/cm et en particulier d'environ 10 mS/cm.

### Etape e)

L'étape d) du procédé selon l'invention vise à éliminer les virus, et notamment les petits virus non enveloppés plus résistants aux traitements d'inactivation virale, susceptibles de se trouver dans la composition purifiée d'anticorps ou de protéine de fusion Fc, afin de garantir la sécurité virale du produit pharmaceutique final.

En effet, les traitements classiques d'inactivation virale, et notamment le traitement solvant-détergent ou détergent seul, ont une efficacité limitée concernant les virus non enveloppés, tels que les parvovirus ou le virus de l'hépatite A.

Or, la nanofiltration, qui repose sur un mécanisme d'exclusion en fonction de la taille des particules, est connue pour être efficace sur les virus non-enveloppés. Les filtres les plus couramment utilisés pour exclure les petits virus non-enveloppés sont les filtres Planova® commercialisés par Asahi Kasei, en particulier les filtres Planova® 15N et Planova® 20N, ayant respectivement une taille moyenne de pores de 15 et 19 nm. Ces filtres, constitués d'une membrane à fibres creuses formée de cellulose régénérée au cuprammonium sont caractérisés par une faible dispersité de la taille des pores (±2 nm autour de la taille moyenne). Cependant, ces filtres sont très coûteux et ne permettent pas le traitement d'une forte charge protéique dans un laps de temps restreint (ex : temps de traitement acceptable de 4 heures), à moins d'augmenter considérablement la surface de filtration (et donc *in fine* le coût de cette étape).

Dans le cadre de la présente invention, les inventeurs ont mis en évidence qu'il est très avantageux d'utiliser un filtre Viresolve® Pro (filtre ayant une double membrane asymétrique de polyéthersulfone retenant au moins 4 logs décimaux de virus ayant une taille d'au moins 20 nm) au lieu d'un filtre Planova® 15N ou Planova® 20N, le filtre Viresolve® Pro permettant de nanofiltrer une charge d'anticorps beaucoup plus importante que les filtres Planova® 15N et Planova® 20N (voir Exemple 4).

L'étape e) consiste donc en une étape de nanofiltration avec un filtre ayant une double membrane de polyéthersulfone d'une porosité d'environ 20 nm.

De tels filtres incluent notamment le filtre Viresolve® Pro (filtre ayant une double membrane asymétrique de polyéthersulfone d'une porosité d'environ 20 nm, commercialisé par Merck-Millipore) et le filtre Virosart® CPV (filtre ayant une double membrane symétrique de polyéthersulfone d'une porosité d'environ 20 nm, commercialisé par Sartorius). La nanofiltration de l'étape e) est avantageusement réalisée en utilisant un filtre ayant une double membrane asymétrique de polyéthersulfone d'une porosité d'environ 20 nm, tel que le filtre Viresolve® Pro commercialisé par Merck-Millipore. Par « une porosité d'environ 20 nm », on entend que la taille moyenne des pores du filtre est comprise entre 17 et 25 nm, avantageusement entre 17 et 24 nm, entre 17 et 23 nm, entre 17 et 22 nm, entre 17 et 21 nm, entre 17 et 20 nm, entre 18 et 25 nm, entre 18 et 24 nm, entre 18 et 23 nm, entre 18 et 22 nm, entre 18 et 21 nm, entre 18 et 20 nm, entre 19 et 25 nm, entre 19 et 24 nm, entre 19 et 23 nm, entre 19 et 22 nm, entre 19 et 21 nm, entre 19 et 20 nm, entre 20 et 25 nm, entre 20 et 24 nm, entre 20 et 23 nm, entre 20 et 22 nm, ou entre 20 et 21 nm.

Dans un mode de réalisation avantageux, l'étape e) comprend entre outre une étape préalable de filtration à travers un filtre en profondeur comprenant des fibres de cellulose, de la terre de diatomée et une résine chargée négativement (pré-filtre Viresolve PreFilter ou VPF) ou une membrane de polyéthersulfone d'une porosité de 0,22 |jm fonctionnalisée par des groupements SO₃⁻ (pré-filtre Viresolve pro Shield notamment).

### Etapes optionnelles

Le procédé selon l'invention peut en outre comprendre une étape d'ultrafiltration et/ou de diafiltration, qui peut se situer soit entre l'étape d) de chromatographie échangeuse d'anions et l'étape e) de nanofiltration, soit après l'étape e) de nanofiltration. Une telle étape peut notamment être réalisée en utilisant des cassettes de type centramate 50 kDa (commercialisées par Pall) ou Pellicon 2 (commercialisées par Merck Millipore) avec un tampon de dialyse comprenant du polysorbate 80 dans le cas où l'ultrafiltration se situe après l'étape e) de nanofiltration.

De plus, une ou plusieurs étapes de filtration stérilisante à travers des filtres ayant une porosité de l'ordre de 0,1 à 0,5 µm (notamment d'environ 0,2 µm) peuvent être présentes à différents stades du procédé selon l'invention. Ces étapes peuvent notamment être réalisées en utilisant un filtre Millipak de 0,22 µm.

Les exemples qui suivent visent à illustrer la présente invention.

### EXEMPLES

### Exemple 1 : Optimisation de l'étape a) de chromatographie d'affinité à la protéine A

L'étape de chromatographie d'affinité à la protéine A est une étape indispensable dans la purification d'anticorps, mais est aussi l'étape la plus coûteuse des procédés de purification d'anticorps.

Afin de réduire significativement le coût de cette étape tout en maintenant la pureté et la qualité du produit, les inventeurs ont testés différentes résines de chromatographie d'affinité à la protéine A et différents tampons d'élutions, et mesuré l'influence de la résine et du tampon d'élution sur un certain nombre de paramètres.

### Matériels et méthodes

### Comparaison de quatre résines de chromatographie d'affinité à la protéine A

### Colonnes testées et préparation

Les caractéristiques des colonnes testées sont :

**Tableau 1. Caractéristiques des colonnes testées**

| Colonne | Lot | Volume (mL) | Dimension (cm) | Résine | Taille moyenne des billes |
|---|---|---|---|---|---|
| MabSelect SuRe™ | 10111269 | 4,7 | 4,7 x 0,77 | agarose fortement réticulé | 85 µm |
| Poros GoPure™ | 121004 | 5,655 | 1,2 x 5 | poly(styrène-divinylbenzène) réticulé revêtu polymère polyhyd roxylé réticulé | 45 µm |
| Toyopearl AF-rProtein A-650F | 0022810 | 5,02 | 14,6 x 3 | polymère méthacrylique | 45 µm |
| Amsphere Protein A JWT203 | 10000006-C03 | 5 | 11,3x 5 | polymère méthacrylique | 49 µm |

Les colonnes sont sanitisées selon la séquence suivante :

**Tableau 2. Séquence de sanitisation des colonnes**

| Solution | Volume colonne (VC) | Débit (mL/min) |
|---|---|---|
| Eau Purifiée | 2 | 3 |
| NaOH 0,5 M | 5 | 3 puis 30 min de temps de contact |
| Eau Purifiée | Qsp pH <8,0 | 3 |
| NaCl 2M | 5 | 3 |

### Détermination du point de "Breakthrough"

Une solution d'anticorps décongelée filtrée 0,2 µm à 2,3 g/L est injectée dans l'appareil de chromatographie (Akta Basic) sans passer par la colonne. La DO 280 nm ainsi lue correspond à la DO 280nm maximale. Cette dernière est de 664 mAU. Le point correspondant à 10% d'une perte de charge de la colonne, appelé « Breakthrough » (10%BT), est ainsi déterminée à 66,4 mAU. La cellule UV ainsi que le circuit de l'appareil sont ensuite rincés en eau puis en tampon d'équilibrage.

### Détermination de la capacité dynamique de fixation à 10% de passage (DBC_{10%BT})

Une fois connectée, la colonne est équilibrée avec du Tampon A (Tris 25 mM ; NaCl 25 mM ; EDTA 5 mM ; pH7,1). Lorsque la colonne est équilibrée, une étape de « pump wash » est effectuée avec la solution d'anticorps pour remplir les tuyaux en amont de la colonne. Quelle que soit la colonne, la solution d'anticorps décongelée filtrée 0,2 µm est injectée à un débit de 3 mL/min (soit un temps de résidence d'environ 1,6 min) et un suivi de la DO 280nm est réalisé.

### Comparaison de quatre tampons d'élution

Quatre tampons d'élution de la chromatographie d'affinité à la protéine A ont été testés à différentes concentrations et différents pH, et leur impact sur l'aspect de l'éluat et sur le pourcentage d'IgG monomérique (et donc sur la présence d'agrégats) a été analysé.

### Composition des tampons testés

Les quatre tampons testés sont les suivants :

**Tableau 3. Tampons testés**

| Tampon | Composition | Concentrations testées | pH testés |
|---|---|---|---|
| Citrate | Citrate trisodique dihydraté | 5 à 25 mM | 2,6 à 3,6 |
| Maleate | Acide maléique, NaOH 0,5 M | 5 à 25 mM | 2,6 à 3,6 |
| Acetate | Acétate de sodium dihydraté | 5 à 25 mM | 2,6 à 3,6 |
| Formiate | Formiate de sodium | 5 à 25 mM | 2,6 à 3,6 |

### Analyse de l'aspect de l'éluat

Une fois l'éluat neutralisé, une analyse visuelle est réalisée par l'opérateur avec l'échelle d'appréciation suivante: 0- Aspect limpide; 1- Trouble léger apparent; 2-Trouble moyen ; 3- Trouble fort (Opalescence). L'éluat neutralisé est ensuite conservé à température ambiante durant 1 heure. Une deuxième observation est menée par le même opérateur selon la même échelle d'appréciation.

### Analyse du pourcentage d'IgG monomérique

Un volume de 500 µL issu de l'éluat neutralisé est analysé par HPLC-SEC (High Performance Liquid Chromatography - Size Exclusion Chromatography) sur une colonne de Superose 12. Les pics générés par lecture de la densité optique à 280 nm sont intégrés par le logiciel Breeze et les aires transformées en pourcentages.

### Résultats

### Comparaison de quatre résines de chromatographie d'affinité à la protéine A

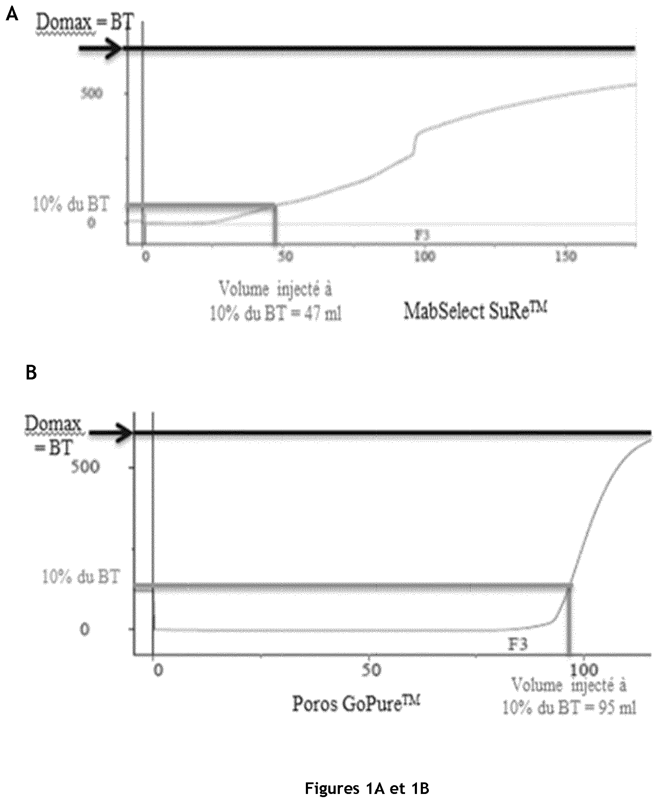

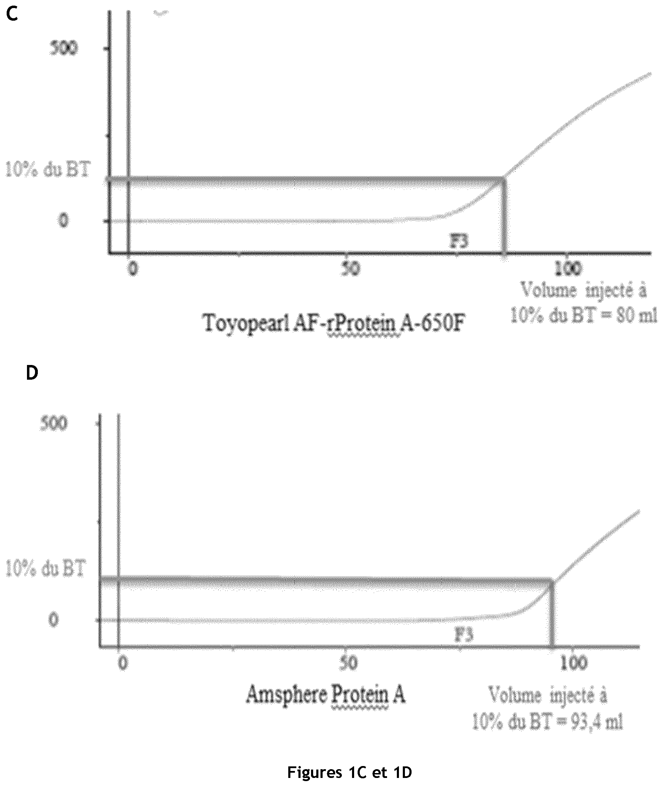

Les résultats obtenus pour les quatre colonnes testées sont présentés sur la **Figure 1****.**

La **Figure 1A** montre que pour la colonne MabSelect SuRe™, un volume de 47 mL a été injecté sur la colonne jusqu'à l'obtention d'une DO 280nm de 66,4 mAU. La capacité de fixation de colonne à 10% du BT (DBC_{10%BT}) est donc de 23 mg/mL de gel MabSelect SuRe™.
La **Figure 1B** montre que pour la colonne Poros GoPure™, un volume de 95 mL a été injecté sur la colonne jusqu'à l'obtention d'une DO 280nm de 66,4 mAU. La capacité de fixation de colonne à 10% du BT (DBC_{10%BT}) est donc de 38,64 mg/mL de gel Poros GoPure™.
La **Figure 1C** montre que pour la colonne Toyopearl AF-rProtein A-650F, un volume de 80 mL a été injecté sur la colonne jusqu'à l'obtention d'une DO 280nm de 66,4 mAU. La capacité de fixation de colonne à 10% du BT (DBC_{10%BT}) est donc de 36,65 mg/mL de gel Toyopearl AF-rProtein A-650F.
La **Figure 1D** montre que pour la colonne Amsphere™ Protein A JWT203, un volume de 93,4 mL a été injecté sur la colonne jusqu'à l'obtention d'une DO 280nm de 66,4 mAU. La capacité de fixation de colonne à 10% du BT (DBC_{10%BT}) est donc de 42,95 mg/mL de gel Amsphere™ Protein A JWT203.

Le **Tableau 4** ci-dessous synthétise les données obtenues avec les quatre colonnes et montre que les colonnes Poros GoPure™ (matrice de poly(styrène-divinylbenzène) réticulé revêtu polymère polyhydroxylé réticulé), Toyopearl AF-rProtein A-650F (matrice de polymère méthacrylique), et Amsphere™ Protein A JWT203 (matrice de polymère méthacrylique) acceptent une charge en anticorps significativement plus élevée que la colonne MabSelect SuRe™ (matrice d'agarose fortement réticulé).

**Tableau 4. Charge en anticorps acceptée par les différentes colonnes testées.**

| Colonne | Volume injecté à 10% de DO max (10%BT) | DBC_{10%BT} (en mg/ml de gel) | 90% du DBC_{10%BT} (en mg/ml de gel) |
|---|---|---|---|
| MabSelect SuRe™ | 47 | 23 | 21 |
| Poros GoPure™ | 95 | 38,64 | 35 |
| Toyopearl AF-rProtein A-650F | 80 | 36,65 | 33 |
| Amsphere™ Protein A JWT203 | 93,37 | 42,95 | 39 |

Afin de confirmer la charge maximum des trois colonnes permettant la plus forte charge en anticorps, du surnageant clarifié filtré a été injecté sur chaque colonne avec une charge en anticorps égale à 90% de la valeur de DBC_{10%BT}. Les charges ainsi appliquées sont les suivantes :

**Tableau 5. Charges appliquées aux différentes colonnes**

| Colonne | 90% du DBC_{10%BT} |
|---|---|
| Poros GoPure™ | 35 mg/mL |
| AF-rProtein A-650F | 33 mg/mL |
| Amsphere™ Protein A JWT203 | 39 mg/mL |

Deux essais ont été réalisés pour chacune des trois colonnes testées.

Les résultats obtenus sont synthétisés dans le **Tableau 6** ci-dessous et montrent que la colonne Amsphere™ Protein A JWT203 donne les meilleurs résultats, aussi bien en termes de charge d'anticorps que de pureté, d'aspect de l'éluat, et même de pH de l'éluat (celui-ci étant ensuite ajusté à un pH d'environ 6,0 pour la suite du procédé). Le rendement de cette colonne est par ailleurs similaire à celui des autres colonnes.
Bien que la charge d'anticorps accepté par la colonne Toyopearl AF-rProtein A-650F soit légèrement plus faible que celle de la colonne Amsphere™ Protein A JWT203, celle-ci permet néanmoins une charge d'anticorps supérieure à 30 mg/mL de gel et donne des résultats satisfaisants en termes de pureté, d'aspect de l'éluat, et de pH de l'éluat. Bien que permettant une charge d'anticorps élevée (au moins 35 mg/mL de gel), la colonne Poros GoPure™ donne quant à elle lieu à un mauvais comportement des éluats qui sont tous apparus troubles. De plus, les résultats sont moins bons en termes de rendement et de pureté.

**Tableau 6. Comparaison de trois colonnes de chromatographie d'affinité à la protéine A**

| Colonne | Injection* (mg/ml de gel) | Essai | Volume éluat | Aspect de l'éluat | pH de l'éluat | Rendement | Pureté |
|---|---|---|---|---|---|---|---|
| Poros GoPure™ | 35 mg/mL | 1 | 3.3 | Trouble | 3.52 | 95,23 | 83,95% |
| | | 2 | 2.7 | Très trouble | 3.75 | 89,2 | 92,52% |
| Toyopearl AF-rProtein A-650F | 33 mg/mL | 1 | 3.2 | Limpide | 3.82 | 94,48 | 94,00% |
| | | 2 | 2.2 | Opalescent | 4.88 | 100 | 93,13% |
| Amsphere™ Protein A JWT203 | 39 mg/mL | 1 | 3.2 | limpide | 4.36 | 91.96 | 95,48% |
| | | 2 | 3 | limpide | 4.78 | 97.5 | 95,88% |

| | | | | | | | |
|---|---|---|---|---|---|---|---|
| * Charge correspondant à 90% de la DBC (Dynamic Binding Capacity) à 10% du BT (Breakthrough) | | | | | | | |

Par comparaison avec le gel MabSelect SuRe™ couramment utilisé dans la première étape de purification d'anticorps par chromatographie d'affinité à la protéine A, le gel Amsphere™ Protein A JWT203 se présente comme un produit ayant un prix au litre deux fois moins cher et une capacité de charge environ deux fois supérieure. Cette colonne permet donc de diminuer par quatre le coût de la première étape de purification par chromatographie d'affinité à la protéine A.

### Comparaison de quatre tampons d'élution

Les résultats concernant l'aspect de l'éluat sont présentés sur la **Figure 2** et montrent que les tampons maléate et formiate permettent d'obtenir un éluat neutralisé stabilisé limpide ou légèrement opalescent pour des concentrations comprises entre 5 et 10 mM (particulièrement à 5 mM) et un pH compris entre 2,6 et 3,6.
Le tampon acétate ne permet pas d'obtenir des éluats limpides et peut même conduire, à faible pH et molarité moyenne à des éluats fortement troublés 1 heure après neutralisation. Quant au tampon citrate, les résultats sont très mauvais en termes d'aspect pour l'éluat neutralisé stabilisé.

Les résultats concernant le pourcentage de formes monomériques de l'anticorps dans l'éluat neutralisé sont présentés sur la **Figure 3**, et montrent que plus de 98% des anticorps sont sous forme monomérique dans l'éluat neutralisé après élution par un tampon citrate ou formiate à une molarité de 5 mM et à un pH entre 2,6 et 3,6.

Bien que permettant d'obtenir des éluats limpides ou seulement faiblement opalescents, le tampon maléate conduit à une formation significative d'agrégats d'anticorps (toujours plus de 5%), ce qui n'est pas souhaitable. Quant au tampon acétate, les résultats sont moins bons qu'avec un tampon citrate ou formiate.

Au total, le tampon donnant les meilleurs résultats, aussi bien en termes d'aspect de l'éluat neutralisé que de pourcentage de formes monomériques d'anticorps dans l'éluat neutralisé est le tampon formiate, utilisé de préférence à une molarité de 5 à 10 mM (de préférence 5 mM) et à un pH entre 2,6 et 3,6 (notamment à un pH de 3,1).

### Conclusions

Par comparaison avec le gel MabSelect SuRe™ couramment utilisé dans la première étape de purification d'anticorps par chromatographie d'affinité à la protéine A, les inventeurs ont pu sélectionner une colonne permettant de diminuer par quatre le coût de la première étape de purification par chromatographie d'affinité à la protéine A, tout en maintenant la pureté et la qualité du produit purifié.

De plus, les inventeurs ont également sélectionné un tampon d'élution particulièrement intéressant pour garantir un éluat neutralisé limpide ou faiblement opalescent et comprenant une très forte majorité de formes monomériques de l'anticorps.

La combinaison spécifique de colonne et de tampon sélectionnée par les inventeurs permet ainsi de diminuer fortement le coût de la purification tout en garantissant un produit de forte pureté et qualité.

### Exemple 2 : Optimisation de l'étape c) de chromatographie échangeuse de cations

Un éluat viralement inactivé issu de l'étape b) du procédé a été purifié par chromatographie échangeuse de cations sur deux types de colonnes distincts : une colonne SP Sepharose et une colonne Capto™ S.

### Matériels et méthodes

### Chromatographie échangeuse de cations sur SP Sepharose®

Un éluat viralement inactivé issu de l'étape b) du procédé a été ajusté à 50 mosm/kg et pH 7,2, et a été injecté sur une colonne SP Sepharose® à une charge d'environ 30,5 g/L de gel selon la séquence :

**Tableau 7. Séquence de purification d'un éluat viralement inactivé issu de l'étape b) du procédé selon l'invention par chromatographie échangeuse de cations sur SP Sepharose®**

| **Dénomination** | **Solution / Tampon utilisé** | **Débit** | **Volume minimum de tampon** | **Paramètres contrôlés** |
|---|---|---|---|---|
| Elimination de la solution de stockage | Eau purifiée | ≤ 200 cm/h | 2VC | NA |
| Equilibrage | Tampon Phosphate de sodium 20 mM ; pH 7,2 ; 50 mosm/kg | ≤ 200 cm/h | 5VC | pH et osmolalité |
| Injection | Produit inactivé ajusté à 50 mosm/kg et pH 7,2 | ≤ 200 cm/h | Environ 5 litres | NA |
| Retour à la ligne de base | Tampon Phosphate de sodium 20 mM ; pH 7,2 ; 50 mosm/kg | ≤ 200 cm/h | 5VC | DO |

| **Inversion du flux sur la colonne** | | | | |
|---|---|---|---|---|
| Elimination du solvant - détergent | Tampon Phosphate de sodium 20 mM ; pH 7,2 ; 50 mosm/kg | ≤ 200 cm/h | 14VC | NA |
| Elution | Tampon Phosphate de sodium 20 mM ; NaCl 150 mM ; pH 7,2 ; 50 mosm/kg | ≤ 200 cm/h | 4VC | Récolte à DO ≥ 0,2AU |

### Chromatographie échangeuse de cations sur Capto™ S

### 1^{er} essai, en parallèle avec SP Sepharose®

Un éluat viralement inactivé issu de l'étape b) du procédé a été ajusté à 5,09 mS/cm grâce à l'ajout de tampon Acétate de sodium 5mM pH6.0, et à pH 5,98, et a été injecté sur une colonne Capto™ S à une charge d'environ 67,3 g/L de gel selon la séquence :

**Tableau 8. Séquence de purification d'un éluat viralement inactivé issu de l'étape b) du procédé selon l'invention par chromatographie échangeuse de cations sur Capto™ S**

| Dénomination | Solution / Tampon | Débit | Volume minimum de tampon | Paramètres contrôlés |
|---|---|---|---|---|
| Elimination de la solution de stockage | Eau purifiée | 8 mL/min Soit 240 cm/h Tps de résidence de 3 min | 2VC | NA |
| Equilibrage | Acétate de sodium 20mM ; NaCl qsp conductivité 5mS/cm; pH 6,0 | | Jusqu'à équilibrage | pH et conductivité |
| Injection | Eluat Protéine A inactivé | | 365 mL (67,3 g/L) | DO 280 nm |
| Retour à la ligne de base | Acétate de sodium 20mM ; NaCl qsp conductivité 5mS/cm; pH 6,0 | | 2VC | DO 280 nm |

| Sens d'injection | Inversion du flux | | | |
|---|---|---|---|---|
| Lavage | Acétate de sodium 20mM ; NaCl qsp conductivité 5mS/cm; pH 6,0 | 8 mL/min Soit 240 cm/h | 14VC | DO 280nm |
| Elution | Tris 20mM ; NaCl qsp conductivité 18mS/cm; pH 7,0 | | 10VC | Récolte à 250 mAu DO 280 nm |

### 2^{ème} essai

Un éluat viralement inactivé issu de l'étape b) du procédé a été ajusté à 5,04 mS/cm grâce à l'ajout de 103,8 mL d'eau purifiée et à pH 6,02 par addition de NaOH 0,5 M. Pour cette étape, du gel de Capto S est compressé dans une colonne de 1 cm de diamètre obtenant ainsi un volume de colonne de 4,8 mL pour une hauteur de 6,1 cm. L'injection sur la colonne échangeuse de cations Capto S s'est déroulée comme suit :

**Tableau 9. Séquence de purification d'un éluat viralement inactivé issu de l'étape b) du procédé selon l'invention par chromatographie échangeuse de cations sur Capto™ S dans un deuxième essai.**

| **Dénomination** | **Solution / Tampon** | **Débit** | **Volume minimum de tampon / solution** | **Paramètres contrôlés** |
|---|---|---|---|---|
| Elimination de la solution de stockage | Eau purifiée | ≤ 600 cm/h | 2VC | NA |
| Equilibrage | Phosphate de sodium 20mM ; NaCl qsp conductivité 5mS/cm; pH 6,0 | ≤ 600 cm/h | Jusqu'à équilibrage | pH et conducti. |
| Injection | Eluat Protéine A | ≤ 600 cm/h | Selon la [ ] en IgG | DO 280nm |
| Retour à la ligne de base | Phosphate de sodium 20mM ; NaCl qsp conductivité 5mS/cm; pH 6,0 | ≤ 600 cm/h | 2VC | DO 280 nm |

| **Sens d'injection** | **Inversion du flux** | | | |
|---|---|---|---|---|
| Lavage | Phosphate de sodium 20mM ; NaCl qsp conductivité 5mS/cm; pH 6,0 | ≤ 600 cm/h | 14VC | DO 280nm |
| inversion du flux, passage en downflow | | | | |
| Elution | Phosphate de sodium 20mM ; NaCl qsp conductivité 18mS/cm; pH 6,9 | ≤ 400 cm/h | 10VC | Récolte à 250mAu DO 280 nm |

La colonne est chargée à raison de 120 grammes d'IgG/L de gel afin de déterminer la capacité maximale de fixation du gel de Capto S.

### Détermination du point de "Breakthrough" et de la capacité dynamique de fixation à 10% de passage (DBC_{10%BT})

Un éluat viralement inactivé issu de l'étape b) du procédé a été ajusté à 5,05 mS/cm et à pH 6,04 par addition d'une solution HCl 6N et d'EPA (Eau Purifiée Apyrogène).

Pour cette étape, du gel de Capto S est compressé dans une colonne de 0.5 cm de diamètre obtenant ainsi un volume de colonne de 3.8 mL pour une hauteur de 19,5 cm. La solution d'anticorps est ensuite injectée dans l'appareil de chromatographie (Akta Basic) sans passer par la colonne. La DO 280nm ainsi lue correspond à la DO 280nm maximale. La cellule UV ainsi que le circuit de l'appareil sont ensuite rincés en eau puis en tampon d'équilibrage.

La capacité dynamique de fixation à 10% de passage (DBC_{10%BT}) a ensuite été déterminée pour trois temps de résidence (1, 2 et 3 minutes) en injectant sur la colonne la solution d'anticorps à différents débits (3,8 mL/min ; 1,9 mL/min ; et 1,3 mL/min, respectivement) et en suivant la DO 280.

### Résultats

### Comparaison chromatographie échangeuse de cations sur SP Sepharose® et chromatographie échangeuse de cations sur Capto™ S

SP Sepharose® : le volume d'éluat recueilli est de 150 mL, avec une concentration protéique estimée de 25.96 g/L ainsi qu'une osmolalité de 273mosm/kG et un pH de 7,09.

La quantité d'anticorps présente dans l'éluat était de 3894 mg. Le rendement d'étape est de 88.6%. Le produit est limpide avec quelques particules.

Capto™ S: en fin d'élution, un volume de 174 mL est obtenu soit 7.3 VC. La concentration finale de l'éluat est de 9,0 g/L et d'aspect limpide en sortie colonne. Le rendement d'étape est alors de 94,3% et le pH et la conductivité de l'éluat sont respectivement de 6,66 et 17,11 mS/cm.

L'éluat est ensuite filtré sur une capsule Millipak 20 de 0,22 µm préalablement conditionnée avec du tampon Tris 20mM ; NaCl qsp conductivité 18 mS/cm; pH 7,0. La filtration est effectuée avec la pompe « pomp L02 » à une vitesse de 30 rpm via un tuyau 184. Le filtre est ensuite rincé avec du tampon Tris 20mM ; NaCl qsp conductivité 18mS/cm; pH 7,0. A l'issue de cette filtration, un volume de 208,8 mL est obtenu avec une concentration de 6,8 g/L et un rendement d'étape de filtration de 90,7%. La pureté de l'éluat filtré est de 99,6% (en protéines).

**Tableau 10. Données comparatives pour la purification par chromatographie échangeuse de cations sur SP Sepharose® et Capto™ S.**

| Paramètre analysé | SP Sepharose® | Capto™ S |
|---|---|---|
| Charge en anticorps | 30,5 g/L de gel | 67,3 g/L de gel |
| Volume élué | 150 mL | 174 mL |
| Rendement | 88.6% | 94,3% |
| Aspect de l'éluat | limpide avec quelques particules | limpide |
| Pureté | ND | 99,6% |

Le **Tableau 10** ci-dessus montre que la colonne Capto™ S permet une charge d'anticorps deux fois supérieure à la colonne SP Sepharose® (d'où une réduction des coûts), un meilleur rendement (d'où une réduction des coûts), tout en garantissant un aspect limpide et une très bonne pureté.

### 2^{ème} essai de chromatographie échangeuse de cations sur Capto™ S

Le point d'inflexion de la courbe de DO se trouve à 110mL, la DO 280nm qui jusque-là était de 1650 mAU monte progressivement, traduisant une fuite d'IgG à travers la colonne. Ce qui donne une capacité maximale de fixation d'environ 84 g/L.

En fin d'élution, un volume de 57,2 mL est obtenu soit 11,9 VC. L'élution est dans un premier temps très rapide mais traine ensuite dans le temps (redescente très lente de la DO280nm). La concentration finale de l'éluat est de 7,30 g/L et d'aspect limpide en sortie colonne. En revanche, des particules apparaissent au bout de quelques minutes. Le rendement d'étape, si l'on se base sur les 110 mL de produit injecté, est de 100%. De plus, après chromatographie échangeuse de cations sur gel Capto S, la solution d'IgG apparaît pure à 100% (en protéines).

Ainsi, ce 2^{ème} essai confirme que la charge maximale en anticorps de la colonne Capto™ S est largement supérieure à celle de la colonne SP Sepharose®.

### Point de "Breakthrough" (BT) et capacité dynamique de fixation à 10% de passage (DBC_{10%BT}) de la colonne Capto™ S

La DO 280nm maximale - lue en injectant la solution d'anticorps dans l'appareil de chromatographie (Akta Basic) sans passer par la colonne - est de 535 mAU. Le point correspondant à 10% d'une perte de charge de la colonne, appelé « Breakthrough » (10%BT), est ainsi déterminé à 54 mAU.

La mesure de la capacité dynamique de fixation à 10% de passage (DBC_{10%BT}) pour des temps de résidence de 1, 2 et 3 minutes a donné les résultats présentés dans le **Tableau 11** suivant :

**Tableau 11. Résultats de mesure de la capacité dynamique de fixation à 10% de passage (DBC_{10%BT}) pour des temps de résidence de 1, 2 et 3 minutes.**

| Temps de résidence (débit) | Volume injecté à 10% de DO max (10%BT) | DBC_{10%BT} (en g/L de gel) | 90% du DBC_{10%BT} (en g/L de gel) |
|---|---|---|---|
| 1 minute (3,8 mL/min) | 207,8 | 87 | 78 |
| 2 minutes (1,9 mL/min) | 250.5 | 104 | 94 |
| 3 minutes (1,3 mL/min) | 265.6 | 111 | 100 |

Ces résultats confirment la capacité de fixation bien supérieure de la colonne Capto™ S par rapport à la colonne SP Sepharose®.

### Conclusions

La sélection par les inventeurs de la colonne Capto™ S pour l'étape c) de chromatographie échangeuse de cations, à la place de la colonne SP Sepharose® couramment utilisée dans cette étape de purification d'un anticorps, permet là encore de diminuer les coûts de purification.

### Exemple 3 : Optimisation de l'étape e) de nanofiltration

L'étape e) de nanofiltration est essentielle pour garantir la sécurité virale de la composition d'anticorps finale, en particulier vis-à-vis des petits virus non-enveloppés. Toutefois, cette étape est également une étape très coûteuse, les nanofiltres étant des produits très chers.

Chaque nanofiltre n'étant utilisé qu'une seule fois, les inventeurs ont testé plusieurs nanofiltrée distinct à fin d'optimiser la charge d'anticorps susceptible d'être traitée en une seule fois, de façon à diminuer le coût de cette étape.

De plus, l'intérêt d'utiliser une préfiltre avec une porosité plus importante pour augmenter la charge en anticorps a également été étudié.

### Matériels et méthodes

### Comparaison de trois filtres distincts

### Produit de départ

Le filtrat mustang Q (étape d)) est dilué au 2/3 a l'aide d'un tampon Citrate trisodique dihydraté 22,05g/L ; NaCl 18,23 g/L ; pH 6,5 ; 800 mosm/kg soit un volume final de 555mL. On réalise ensuite une filtration 0,2µm sur un filtre Millipak 40 de 0,02m2 suivi d'un rinçage au tampon K soit un volume final de 672mL, une concentration de 5,28g/L, un pH de 7,14 et une osmolalité de 367 mosm/kg.

Le produit est ensuite filtré sur un filtre Pall en PVDF hydrophile de grade 0,1 µm, Suivi d'un rinçage au tampon K soit un volume final de 643mL, une concentration de 5,16g/L., un pH de 7,12 et une osmolalité de 366 mosm/kg.

### Filtres utilisés

Les caractéristiques des nanofiltres testés sont les suivantes :

**Tableau 12. Caractéristiques des filtres testés**

| Filtre | Fabricant | Membrane | Porosité |
|---|---|---|---|
| Planova® 15N | Asahi Kasei | fibres creuses de cellulose régénérée au cuprammonium | 15 ± 2 nm |
| Planova® 20N | | | 19 ± 2 nm |
| Viresolve pro 20N | Millipore | double membrane asymétrique de polyéthersulfone | Environ 20 nm |

### Nanofiltration sur Planova® 15N

L'étape de nanofiltration est réalisée sur filtre Planova® 15N de 0,001m² équilibré en tampon Citrate trisodique 7,35g/L ; NaCl 9 g/L ; pH 6,5 ; 360 mosm/kg à une pression de 300 ± 50 mbars. Le débit moyen était de 0.15mL/min. Le filtrat était limpide en fin de nanofiltration.

### Nanofiltration sur Planova® 20N

L'étape de nanofiltration est réalisée sur filtre Planova® 20N de 0,001m² équilibré en tampon Citrate trisodique 7,35g/L ; NaCl 9 g/L ; pH 6,5 ; 360 mosm/kg à une pression de 800 ± 50 mbars.

### Nanofiltration sur Viresolve pro 20N

L'étape de nanofiltration est réalisée sur filtre Viresolve pro 20N de 3,1 cm² équilibré en tampon citrate trisodique 7,35 g/L ; NaCl 9 g/L ; pH 6,5 ; 360 mosm/kg à une pression de 2 bars.

### Validation de l'utilisation du filtre Viresolve pro 20N

Le filtre Viresolve pro 20N a été testé sur plusieurs compositions d'anticorps purifiées, afin de valider la charge en anticorps susceptible d'être filtrée en une seule fois.

### Essai 1

La totalité de l'éluat de chromatographie échangeuse de cations (étape c) du procédé selon l'invention) a été filtré sur une capsule Mini Kleenpak de 0,22 µm avec un rinçage du filtre à l'aide de tampon phosphate 20 mM ; pH 6,9 ; conductivité 5 mS/cm. A l'issue de cette filtration, un volume de 64,3 mL est obtenu avec une concentration de 5,9 g/L. Le rendement de filtration est de 90,7%, celui-ci s'explique par le fait que le filtre a été rincé avec peu de tampon pour éviter de descendre trop bas en concentration d'IgG pour l'étape suivante de nanofiltration. Le produit est stable après filtration 0,22 µm. Il est ensuite stocké 24h à +4°C.

Les 64,3 mL de matériel départ ont été injectés à 2 bars sur le nanofiltre Viresolve Pro+ de 3,1 cm² équilibré en tampon Phosphate de sodium 20 mM ; conductivité de 5 mS/cm ; pH 6,9. L'aspect du produit stocké 24h à +4°C avant nanofiltration était limpide. L'étape de nanofiltration a donc été réalisée directement sur le produit non filtré sur 0,1 µm au préalable.

### Essai 2

L'aspect du produit issu de la chromatographie échangeuse d'anion sur Mustang Q (étape d) du procédé selon l'invention), soumis à une étape de dialyse, et stocké 24h à +4°C était limpide. Avant nanofiltration, ce dernier a été filtré sur une capsule Mini Kleenpak de 0,1 µm avec un rinçage du filtre à l'aide de tampon Citrate trisodique dihydraté 8,82 g/L, NaCl 3,25 g/L ; Mannitol 17 g/L ; pH 6,5 ; 300 mosm/kg. A l'issue de cette filtration, un volume de 264 mL est obtenu avec une concentration de 5,1 g/L. Le rendement de filtration est de 100%.

La solution dialysée filtrée est injectée à 2 bars sur le nanofiltre Viresolve® Pro de 3,1 cm² équilibré préalablement en tampon Citrate trisodique dihydraté 8,82 g/L, NaCl 3,25 g/L ; Mannitol 17 g/L ; pH 6,5 ; 300 mosm/kg.

### Essai 3

L'éluat Mustang Q® (étape d) du procédé selon l'invention) est injecté à 2 bars sur le nanofiltre Viresolve® Pro de 3,1 cm² équilibré préalablement en tampon Tris 20 mM ; pH 6,5 ; 10 mS/cm.

### Utilisation d'un préfiltre

La nanofiltration d'un même éluat Mustang® Q (étape d) du procédé selon l'invention) directement sur le filtre Viresolve® Pro, ou après passage par un préfiltre Sartorius® ou Millipore® a été testée.

### Nanofiltration directe

L'éluat Mustang® Q (étape d) du procédé selon l'invention) est injecté à 2 bars sur le nanofiltre Viresolve® Pro de 3,1 cm² équilibré préalablement en tampon Tris 20 mM ; pH 6,5 ; 10 mS/cm. Cette expérience correspond à l'essai 3 ci-dessus du filtre Viresolve® Pro.

### Nanofiltration après passage par un préfiltre Sartorius®

Un montage en série d'un préfiltre Sartorius® sur le nanofiltre Viresolve® Pro est réalisé L'éluat Mustang® Q (étape d) du procédé selon l'invention) est injecté à 2 bars sur le nanofiltre Viresolve® Pro de 3,1 cm² équilibré préalablement en tampon Tris 20 mM ; pH 6,5 ; 10 mS/cm.

### Nanofiltration après passage par un préfiltre Millipore®

Un montage en série d'un préfiltre Millipore® (optiscale®-40 Viresolve prefilter réf SSPVA40NB9) sur le nanofiltre Viresolve® Pro de 3,1 cm² est réalisé. L'éluat Mustang® Q est injecté à 2 bars sur le nanofiltre Viresolve® Pro de 3,1 cm² équilibré préalablement en tampon Tris 20 mM ; pH 6,5 ; 10 mS/cm.

### Nanofiltration après passage par un préfiltre Millipore® (validation sur un 2^{ème} produit)

Un montage en série d'un préfiltre Millipore (Viresolve Pro réf C2NA74678) sur le nanofiltre Viresolve Pro de 3,1 cm² est réalisé. Le produit issu de la chromatographie échangeuse d'anion sur Mustang Q (étape d) du procédé selon l'invention) et de l'étape de dialyse est injecté à 2 bars sur le montage Viresolve Pro+ de 3,1 cm² équilibré préalablement avec du tampon Citrate trisodique dihydraté 8,82 g/L, NaCl 3,25 g/L ; Mannitol 17 g/L; pH 6,5.

### Nanofiltration après passage par un préfiltre Millipore® (validation sur un 2^{ème} produit)

Un montage en parallèle de deux préfiltres Millipore (Viresolve Pro Shield réf C2NA74678) est amont du nanofiltre Viresolve Pro de 3,1 cm². Le montage est préalablement équilibré dans son intégralité sous une pression de 2 bars avec de l'EPA puis en tampon Citrate trisodique dihydraté 8,82 g/L, NaCl 3,25 g/L ; Mannitol 17 g/L; pH 6,5. Le deuxième préfiltre reste clampé au début de l'injection du produit, celui-ci devant être déclampé en cas de colmatage du premier préfiltre. Ce montage permet de déterminer le Vmax du nanofiltre sans que le préfiltre soit éventuellement limitant.
Débit moyen sur 10 min à l'EPA = 2,18 g/min
Débit moyen sur 10 min en tampon Citrate = 2,52 g/min

### Résultats

### Comparaison de trois filtres distincts

### Nanofiltration sur Planova® 15N

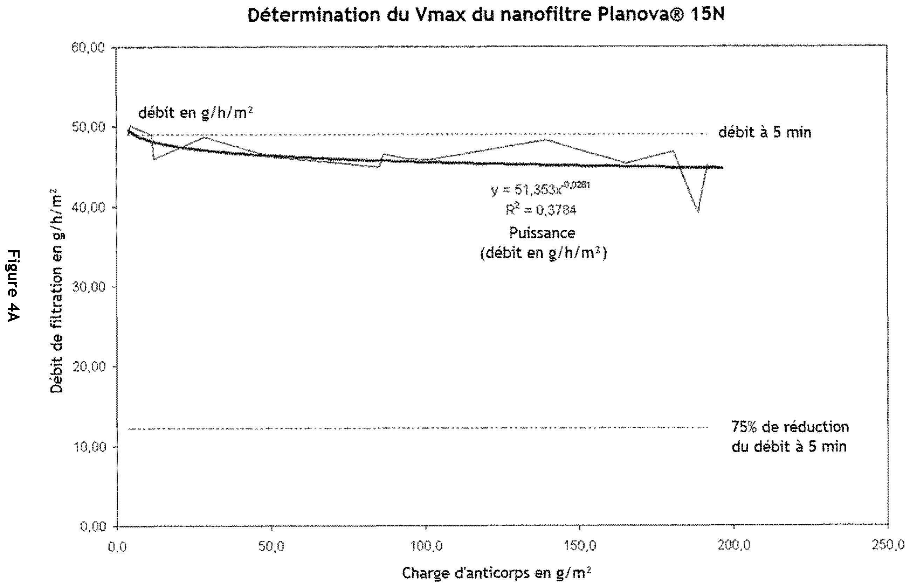

La **Figure 4A** représente le débit de filtration en fonction de la charge d'anticorps. Le débit moyen était de 0,15 mL/min pour une charge en anticorps allant jusqu'à presque 200 g/m². Le filtrat était limpide en fin de nanofiltration.

### Nanofiltration sur Planova® 20N

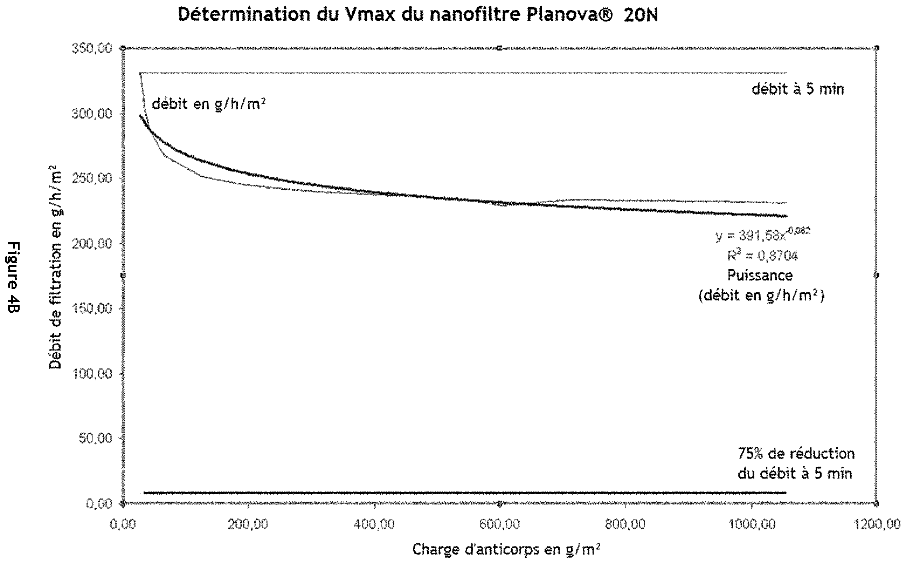

La **Figure 4B** représente le débit de filtration en fonction de la charge d'anticorps. Le débit moyen était de 0,8 mL/min pour une charge en anticorps allant jusqu'à presque 1100 g/m². Le filtrat était limpide en fin de nanofiltration.

### Nanofiltration sur Viresolve® pro 20N

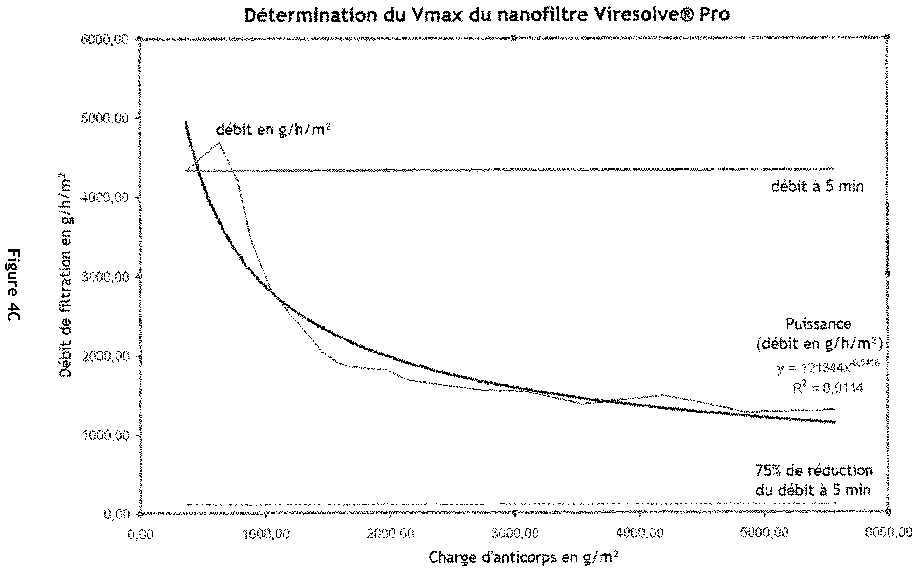

La **Figure 4C** représente le débit de filtration en fonction de la charge d'anticorps. Le débit moyen était de 2,4 mL/min pour une charge en anticorps allant jusqu'à environ 5500 g/m². Le filtrat était limpide en fin de nanofiltration.

### Comparaison de la charge d'anticorps nanofiltrée en fonction du temps de filtration

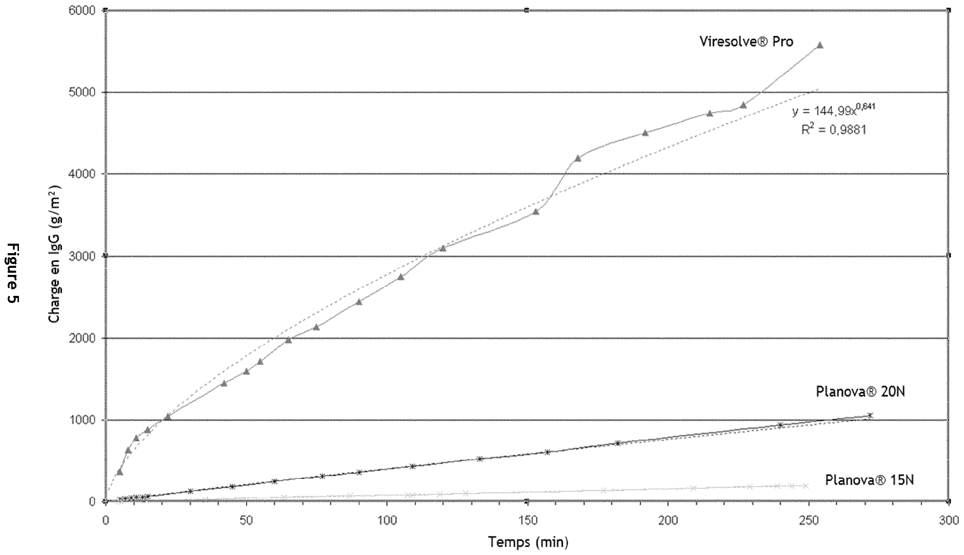

La **Figure 5** représente la charge d'anticorps nanofiltrée en fonction du temps de filtration, et illustre très clairement la forte supériorité du filtre Viresolve® pro 20N par rapport aux filtres Planova® 15N et Planova® 20N.

Par extrapolation on aurait au bout de 4 heures :
Planova® 15N → 200 g d'IgG / m²
Planova® 20N → 1000 g d'IgG / m²
Viresolve® pro+ → 5000 g d'IgG / m²

### Validation de l'utilisation du filtre Viresolve pro 20N

### Essai 1

Un colmatage du filtre Viresolve® Pro+ est noté au bout de 30 min. Une charge de 141 mg de produit a donc pu être nanofiltrée sur 3,1 cm² de surface filtrante soit une quantité d'anticorps de 455 g/m² de nanofiltre. Cette charge est significativement inférieure à celle obtenue précédemment lors de la comparaison des trois filtres Planova® 15N, Planova® 20N et Viresolve® Pro+ (4865 g/m²).

Néanmoins, la diminution peut être liée aux caractéristiques du produit filtré (différent de celui filtré lors de la comparaison des trois filtres) et la charge maximale reste largement supérieure à celle possible avec le filtre Planova® 15N.

### Essai 2

Un colmatage du filtre Viresolve® Pro est noté au bout de 26,41 mL soit une quantité de 135,48 mg d'anticorps et une charge de 437 g/m² de nanofiltre. La charge est d'environ 300 g/m² à V75 et de 425 g/m² à V90. La charge maximale est similaire à celle obtenue à l'essai 1 et confirme l'intérêt du filtre Viresolve® Pro par rapport aux filtres Planova®.

La pureté du produit nanofiltré est déterminée par chromatographie à exclusion de taille (HPSEC). Elle est estimée à 98,83 % à ce stade du procédé.

### Essai 3

La filtration est arrêtée suite au colmatage du nanofiltre après 7,21 mL de produit soit une quantité de 25,96 mg d'anticorps et une charge de 84 g/m². Cette charge maximal est beaucoup plus faible que celles précédemment observées et illustre une possible variabilité de la charge maximale en fonction de l'état du produit à nanofiltrer. Cette variabilité justifie le test de l'utilisation d'un préfiltre.

### Utilisation d'un préfiltre

### Nanofiltration directe

La filtration est arrêtée suite au colmatage du nanofiltre après 7,21 mL de produit soit une quantité de 25,96 mg d'anticorps et une charge de 84 g/m².

### Nanofiltration après passage par un préfiltre Sartorius®

Un colmatage du préfiltre est noté au bout 23,82 mL soit une quantité de 85,75 mg d'anticorps filtré et une charge de 277 g/m². Dans le cas présent, la limite de charge est imposée par le préfiltre. Le préfiltre Sartorius® améliore donc la charge maximale en anticorps, mais ne permet pas de restaurer une très forte charge en anticorps.

### Nanofiltration après passage par un préfiltre Millipore®

Un colmatage du préfiltre est noté au bout de 97 mL soit une quantité de 349,2 mg d'anticorps et une charge de 1126 g/m². Dans le cas présent, la limite de charge est imposée par le préfiltre.

Le préfiltre Millipore® « optiscale®-40 Viresolve® prefilter » améliore donc la charge maximale en anticorps, permettant d'obtenir une charge supérieure à celle déjà élevée des essais de validation 1 et 2 décrits ci-dessus avec le nanofiltre Viresolve® Pro seul.

### Nanofiltration après passage par un préfiltre Millipore® (validation sur un 2^{ème} produit)

Un colmatage du montage est noté au bout de 2 h 25 min après que soient passés 193,2 mL (à 3,23 g/L après filtration 0,22 µm) soit une quantité d'environ 624 mg d'anticorps et une charge de 2013 g/m². Le montage est alors clampé et mise en attente jusqu'au lendemain.

Après remplacement du préfiltre et reprise de la filtration, un colmatage du montage est noté au bout de 24 min avec 14,0 mL soit une quantité de 45,2 mg d'anticorps (charge de 146 g/m²). La charge totale de la solution Viresolve® Pro+ est donc de 2159 g/m². A noter que la déstabilisation du produit durant la nuit ne permet pas de savoir lequel du préfiltre ou du filtre est à l'origine de ce colmatage.

Dans tous les cas, cette expérience valide l'intérêt d'utiliser un préfiltre avant la nanofiltration sur le filtre Viresolve® Pro+, pour garantir une très forte charge en anticorps.

### Nanofiltration après passage par un préfiltre Millipore® (validation sur un 3^{ème} produit)

Les 418,6 mL de produit issus de la dialyse soit 938 mg (3Kg/m²) ont été intégralement nanofiltrés en 217 minutes avec un débit final équivalent à 71,5% du débit initial. Le volume de nanofiltrat obtenu était de 407,5 mL à une concentration de 2,25g/L soit 917 mg de protéines ce qui donne un rendement d'étape sans rinçage du nanofiltre de 97,8%.

Le montage utilisé a permis d'obtenir une charge de 2958 g/m², soit une charge 2,6 fois supérieure à celle du premier essai et 1,4 fois supérieure à celle obtenue avec le 2^{ème} produit.

### Tableau récapitulatif des résultats obtenus

Les différents résultats obtenus sont synthétisés dans le **Tableau 13** suivant :

**Tableau 13. Synthèse des résultats obtenus pour l'étape e) de nanofiltration.**

| Préfiltre | Nanofiltre | Charge en anticorps | Commentaires |
|---|---|---|---|
| Non | Planova® 15N | 200 g/m² | Très faible |
| Non | Planova® 20N | 1100 g/m² | Moyen ne |
| Non | Viresolve® pro 20N | Comparaison 3 filtres : 5500 g/m² | Potentiellement élevée, mais très variable, avec problèmes de colmatage |
| | | Validation : | |
| | | Essai 1 : 455 g/m² | |
| | | Essai 2 : 437 g/m² | |
| | | Essai :3 : 84 g/m² | |
| Sartorius® | Viresolve® pro 20N | 277 g/m² | Colmatage du préfiltre |
| *Millipore*® « optiscale®-40 Viresolve® prefilter » | Viresolve® pro 20N | Essai 1 (1 préfiltre) : 1126 g/m² | Moyenne à élevée, avec variabilité limitée |
| | | Essai 2 (1 préfiltre) : 2159 g/m² | |
| | | Essai 3 (2 préfiltres en parallèle) : 2958 g/m² | |

### Conclusions

Les expériences réalisées par les inventeurs montrent clairement l'intérêt d'utiliser un filtre Viresolve® Pro pour la nanofiltration d'une composition purifiée d'anticorps, afin d'augmenter fortement la charge en anticorps traitée en une seule fois et ainsi diminuer significativement les coûts associés à cette étape particulièrement couteuse. De plus, l'ajout d'un préfiltre permet d'améliorer encore la charge en anticorps traitée en une seule fois.

Au total, par rapport à une étape de nanofiltration utilisant un filtre Planova® 15N et une charge d'anticorps de 50 g/m² (procédé antérieur du déposant), les modifications liées à la sélection du filtre Viresolve® Pro et à l'ajout d'un préfiltre permettent d'obtenir un gain de charge d'un facteur supérieur à 40.

### Exemple 4. Optimisation de l'élimination des impuretés lors de l'étape a) de chromatographie d'affinité à la protéine A

Afin d'optimiser l'élimination des impuretés lors de l'étape a) de chromatographie d'affinité à la protéine A, différentes conditions ont été testées sur une nouvelle colonne avec une résine comprenant une résine à base de polymère de méthacrylate sous forme de billes d'un diamètre moyen d'environ 40-50 µm, sur laquelle est greffée un tétramère de domaine C modifié stable en conditions alkalines produit dans *E. coli* (colonne Amsphere™ Protein A A3, appelée « A3-JSR » dans la suite).

Cette colonne est particulièrement avantageuse puisqu'elle permet d'obtenir des éluats avec un bon rendement, une faible turbidité, et une bonne élimination des impuretés et notamment des autres protéines produites par la cellule de production (appelées « HCP » pour « Host cell proteins »), et de l'ADN de la cellule de production (appelé « HC-DNA » pour « Host cell DNA ») (voir **Tableaux 16 et 17** ci-dessous), et ce avec une charge élevée. En effet, la valeur 90% du DBC_{10%BT} de cette colonne est de 58mg/mL. Notamment, différentes concentrations en NaCl de la solution saline de lavage et différents pH d'élution ont été testés pour la colonne A3-JSR (voir **Tableau 15** ci-dessous).

Les conditions testées pour la colonne A3-JSR sont résumées dans les **Tableaux 14 et 15** ci-dessous :

**Tableau 14. Conditions générales testées pour la colonne A3-JSR.**

| **Dénomination** | **Solution / Tampon utilisé** | **Temps de résidence** | **Volume minimum de tampon / solution** | **Paramètres contrôlés** |
|---|---|---|---|---|
| Equilibrage | Tampon Tris 25mM ; EDTA 5mM; NaCl 25mM pH7.1 | 3 minutes | Jusqu'à équilibrage | pH et conducti. |
| Injection | Surnageant de culture clarifié filtré 0,2µm | | 58mg/ml de gel | DO 280nm |
| Retour Ligne de Base | Tampon Tris 25mM ; EDTA 5mM; NaCl 25mM pH7.1 | | 4VC | NA |
| Lavage (inversion flux upflow) | Cf **Tableau 15** | | 4VC | NA |
| Retour à la Ligne de Base | Tampon Tris 25mM ; EDTA 5mM; NaCl 25mM pH7.1 | | Jusqu'à RLB | DO 280 nm |
| Elution | Tampon Formiate 5mM, Arginine 200mM, pH Cf **Tableau 15** | | 4VC | Récolte à 250mAu DO 280 nm |

**Tableau 15. Force ionique de la solution de lavage et pH de la solution d'élution testés pour la colonne A3-JSR.**

| A3-JSR | | |
|---|---|---|
| Charge : 58mg/ml de gel ; Volume injecté : 35 mL ; Temps de résidence : 3 minutes ; Débit : 0,6 mL/min | | |
| N° Run | [NaCl] dans lavage (mM) | pH d'élution |
| 1A | 700 | 2.6 |
| 2A | 1200 | 3.94 |
| 3A | 1200 | 3.1 |
| 4A | 700 | 3.6 |
| 5A | 1200 | 3.1 |
| 6A | 700 | 3.6 |
| 7A | 1700 | 3.6 |
| 8A | 1700 | 2.6 |
| 9A | 2041 | 3.1 |
| 10A | 700 | 2.6 |
| 11A | 1200 | 3.1 |
| 12A | 1200 | 2.26 |
| 13A | 1200 | 3.1 |
| 14A | 1200 | 3.1 |
| 15A | 359 | 3.1 |
| 16A | 1700 | 3.6 |
| 17A | 1700 | 2.6 |

Les résultats obtenus juste à l'issue de l'élution sont résumés dans le **Tableau** 16 et ceux obtenus après neutralisation dans le **Tableau 17** ci-dessous :

**Tableau 16. Résultats obtenus juste après élution, avant neutralisation.**

| N° Run | [NaCl] dans lavage (mM) | pH d'élution | volume étution | [lg] | Q lg | Rdt | pH | Turbidité |
|---|---|---|---|---|---|---|---|---|
| 1A | 700 | 2.6 | 2.71 | 17.59 | 47.67 | 82.2% | / | 0.01 |
| 2A | 1200 | 3.94 | 9.99 | 3.82 | 38.16 | 65.8% | / | / |
| 3A | 1200 | 3.1 | NA | / | / | / | / | / |
| 4A | 700 | 3.6 | 4.06 | 12.13 | 49.25 | 84.9% | 4.88 | 0.016 |
| 5A | 1200 | 3.1 | 2.59 | 17.65 | 45.71 | 78.8% | 4.09 | 0.036 |
| 6A | 700 | 3.6 | 4.98 | 9.84 | 49.00 | 84.5% | 4.56 | 0.020 |
| 7A | 1700 | 3.6 | 4.68 | 10.69 | 50.03 | 86.3% | 4.57 | 0.022 |
| 8A | 1700 | 2.6 | 2.19 | 16.81 | 36.81 | 63.5% | 5.88 | 0.031 |
| 9A | 2041 | 3.1 | 2.58 | 18.3 | 47.21 | 81.4% | 4.07 | 0.051 |
| 10A | 700 | 2.6 | 3.41 | 16.81 | 57.32 | 98.8% | 3.58 | 0.095 |
| 11A | 1200 | 3.1 | 3.94 | 14.93 | 58.82 | 101.4% | 4.36 | 0.092 |
| 12A | 1200 | 2.26 | 4.04 | 14.18 | 57.29 | 98.8% | 2.81 | 0.114 |
| 13A | 1200 | 3.1 | 2.54 | 15.59 | 39.60 | 68.3% | 3.97 | 0.038 |
| 14A | 1200 | 3.1 | 2.63 | 18.01 | 47.37 | 81.7% | 4.01 | 0.011 |
| 15A | 359 | 3.1 | 2.5 | 18.93 | 47.33 | 81.6% | 4.03 | 0.012 |
| 16A | 1700 | 3.6 | 4.07 | 11.97 | 48.72 | 84.0% | 4.88 | 0.018 |
| 17A | 1700 | 2.6 | 2.66 | 16.01 | 42.59 | 73.4% | 3.05 | 0.023 |

**Tableau 17. Résultats obtenus après élution et neutralisation.**

| N° Run | [NaCl] dans lavage (mM) | pH d'élution | pH | volume tampon | Turbidité | [lg] | HCP ng/mg | Log HCP | ADN pg/mg | Log ADN |
|---|---|---|---|---|---|---|---|---|---|---|
| 1A | 700 | 2.6 | 6.03 | 50 | / | 10.08 | 57.6 | 3.8 | 933 | 3.2 |
| 2A | 1200 | 3.94 | 6.17 | 50 | / | 3.72 | 88.1 | 3.6 | 12166 | 2.0 |
| 3A | 1200 | 3.1 | / | / | Limpide | | | | | |
| 4A | 700 | 3.6 | 6.34 | 10 | Légèreme nt trouble | 6.93 | 84.3 | 3.6 | 603.2 | 3.4 |
| 5A | 1200 | 3.1 | 7.1 | 30 | Limpide | 9.61 | 67.8 | 3.7 | 58 | 4.4 |
| 6A | 700 | 3.6 | 7.02 | 30 | Légèreme nt trouble | 8.53 | 76.6 | 3.6 | 434.3 | 3.5 |
| 7A | 1700 | 3.6 | 6.47 | 15 | Légèreme nt trouble | 8.79 | 92.5 | 3.6 | 12.5 | 5.0 |
| 8A | 1700 | 2.6 | 3.08 | 30 | Légèreme nt trouble | 6.55 | 70.8 | 3.7 | 4.9 | 5.4 |
| 9A | 2041 | 3.1 | 6.55 | 20 | Trouble | 4.68 | 98.5 | 3.5 | 5874 | 2.4 |
| 10A | 700 | 2.6 | 6.04 | 80 | Trouble | 5.58 | 2000 | 2.2 | 28757 | 1.7 |
| 11A | 1200 | 3.1 | 6.43 | 60 | Trouble | 5.68 | 4900 | 1.8 | 9691 | 2.1 |
| 12A | 1200 | 2.26 | 6.84 | 250 | Légèreme nt trouble | 4.55 | 1260 | 2.4 | 15187.7 | 1.9 |
| 13A | 1200 | 3.1 | 7.72 | 50 | | 3.8 | 114.7 | 3.5 | 245.16 | 3.7 |
| 14A | 1200 | 3.1 | 6.86 | 35 | Limpide | 4.79 | 40.5 | 3.9 | 27.55 | 4.7 |
| 15A | 359 | 3.1 | 6.98 | 30 | Limpide | 4.38 | 49.8 | 3.8 | 866.4 | 3.2 |
| 16A | 1700 | 3.6 | 6.5 | 20 | Légèreme nt trouble | 4.28 | 44.2 | 3.9 | 11.45 | 5.1 |
| 17A | 1700 | 2.6 | 6.98 | 70 | Limpide | 3.46 | 46.2 | 3.9 | 3 | 5.7 |

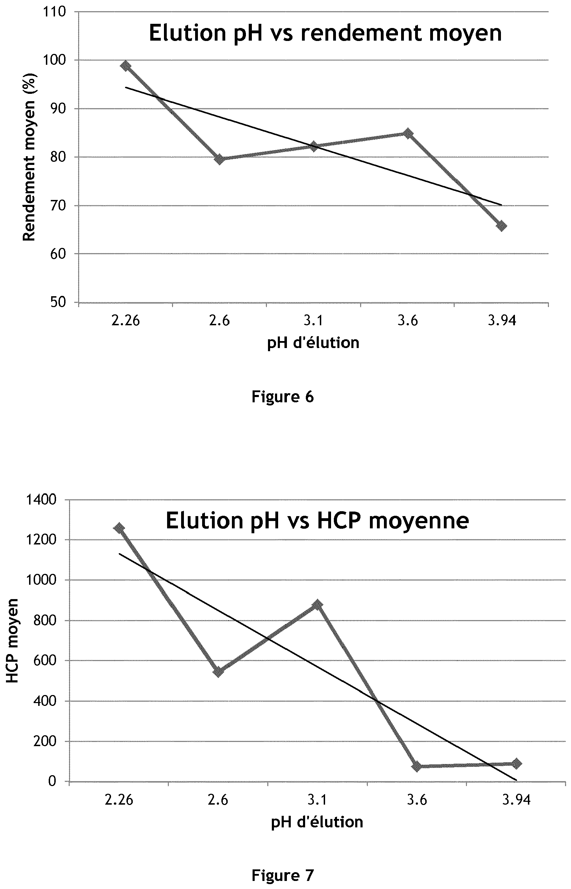

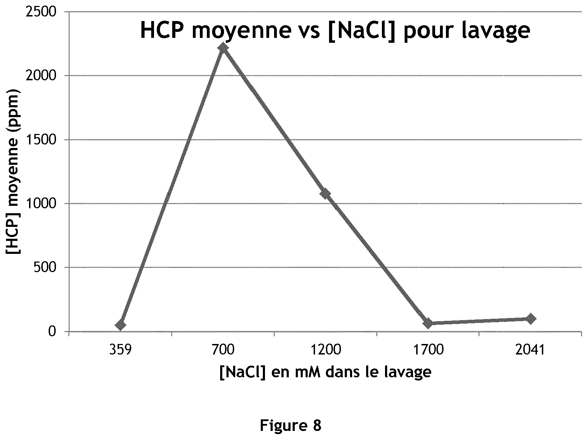

La variation du rendement moyen en fonction du pH d'élution utilisé est représentée sur la **Figure 6****.** De même, la variation de l'élimination moyenne des HCP en fonction de la concentration en NaCl dans la solution de lavage et du pH d'élution sont représentées sur les **Figures 7** **et** **8****.**

Les résultats présentés dans les **Tableaux 16 et 17** ci-dessus et sur les **Figures 6 à 8** montrent que les conditions optimales de rendement correspondent à un pH d'élution entre 2,6 et 3,6, et que les conditions optimales pour l'élimination des HCP correspondent à un pH d'élution entre 3,1 et 4 et à une solution de lavage comprenant au moins 1,2 M de NaCl.

### RÉFÉRENCES BIBLIOGRAPHIQUES

Fahrner RL, Knudsen HL, Basey CD, Galan W, Feuerhelm D, Vanderlaan M, Blank GS. Industrial purification of pharmaceutical antibodies: development, operation, and validation of chromatography processes. Biotechnol Genet Eng Rev. 2001;18:301-27.
Liu HF, Ma J, Winter C, Bayer R. Recovery and purification process development for monoclonal antibody production. MAbs. 2010 Sep-Oct;2(5):480-99.
Edelman, G.M. et al., Proc. Natl. Acad. USA, 63, 78-85 (1969)
Kabat et al., Sequences of Proteins of Immunological Interest, 5th Ed. Public Health Service, National Institutes of Health, Bethesda, Md. (1991).
WO00/42072,
Shields RL, et al. J Biol Chem. 2001 Mar 2;276(9):6591-604.
Lazar, G. A., et al. Proc Natl Acad Sci U S A. 103(11): 4005-10.
WO2004/029207
WO/2004063351
WO2004/074455
WO99/51642
WO2004074455A2
Idusogie EE et al. J Immunol. 2001; 166:2571-5.
Dall'Acqua et al. J Immunol 2006; 177:1129-1138.
Moore GL. Et al. mAbs 2:2, 181-189; March/April, 2010.
Verhoeyn et al. BioEssays, 8 : 74, 1988.
Verhoeyen et al. Science, 239 : 1534-1536, 1988.
Jones et al. Nature, 321 : 522-525, 1986.
Riechmann et al. Nature, 332 : 323-327, 1988.
Almagro et al. Frontiers in Bioscience 13, 1619-1633, January 1, 2008.
Jakobovits et al., Proc. Natl. Acad. Sci. USA. 90:2551 (1993).
Jakobovits et al., Nature, 362:255-258 (1993).
Bruggermann et al., Year in Immuno., 7:33 (1993).
Duchosal et al. Nature 355:258 (1992)US5,591,669
US 5,598,369
US 5,545,806
US 5,545,807
US 6,150,584
Hoogenboom et al., J. Mol. Biol., 227:381 (1991).
Marks et al., J. Mol. Biol., 222:581- 5 597 (1991).
Vaughan et al. Nature Biotech 14:309 (1996).
WO2012/041768
Manipulating the Mouse Embryo, A Laboratory Manual, Second edition, Cold Spring Harbor Laboratory Press (1994).
Gene Targeting, A Practical Approach, IRL Press at Oxford University Press (1993).
Ryan et aL, 1997 Science; 278: 873 - 876.
Cibelli et al., 1998 Science, 280 : 1256-1258.
WO0026357A2
WO9004036A1
WO9517085A1
WO0126455A1
WO2004050847A2
WO2005033281A2
WO2007048077 A2
WO0126455A1
WO2007106078A2
Stoger E, et al. Molecular Breeding 9: 149-158, 2002.
Fisher R, et al. Vaccine 21 (2003) 820-825.
Forthal et al, J Immunol 2010;185;6876-6882.
Ma JK, et al. Nat Rev Genet. 2003 Oct;4(10):794-805.
Schillberg S, et al. Vaccine 23 (2005) 1764-1769.
US6,013,763
US5,084,559
US5,260,373
US6,399,750
US7,709,209
WO2012/083425
Löfdahl S, Guss B, Uhlén M, Philipson L, Lindberg M. Gene for staphylococcal protein A. Proc Natl Acad Sci U S A. 1983 Feb;80(3):697-701.
Uhlén M, Guss B, Nilsson B, Gatenbeck S, Philipson L, Lindberg M. Complete sequence of the staphylococcal gene encoding protein A. A gene evolved through multiple duplications. J Biol Chem. 1984 Feb 10;259(3):1695-702.

## Revendications

1. Procédé de purification d'un anticorps monoclonal ou d'une protéine de fusion entre le fragment Fc d'un anticorps et un second polypeptide, comprenant :
a) une étape de chromatographie d'affinité sur une résine ayant pour matrice un gel de polymère de méthacrylate réticulé, sur lequel est greffée de la protéine A,
b) une étape d'inactivation virale,
c) une étape de chromatographie échangeuse de cations sur une résine ayant pour matrice un gel d'agarose réticulé, sur lequel sont greffés des groupements sulfonate (-SO₃-) par l'intermédiaire de bras espaceurs à base de dextrane,
d) une étape de chromatographie échangeuse d'anions sur une membrane hydrophile de polyéthersulfone revêtue d'un polymère réticulé sur lequel sont greffés des groupements amine quaternaire (Q), et
e) une étape de nanofiltration avec un filtre ayant une double membrane de polyéthersulfone d'une porosité d'environ 20 nm,
**caractérisé en ce que** le tampon d'élution utilisé à l'étape a) pour éluer l'anticorps est un tampon formiate utilisé à une molarité de 5 à 10 mM et à un pH compris entre 2,6 et 3,6.

2. Procédé selon la revendication 1, **caractérisé en ce que** le gel de polymère de méthacrylate réticulé sur lequel est greffée de la protéine A utilisé à l'étape a) est sous forme de billes ayant un diamètre moyen compris entre 30 et 60 µm, avantageusement entre 40 et 50 µm.

3. Procédé selon la revendication 1 ou la revendication 2, **caractérisé en ce que** l'étape a) comprend une sous-étape de lavage de la résine avec une solution saline comprenant une concentration en NaCl d'au moins 1M.

4. Procédé selon l'une quelconque des revendications 1 à 3, **caractérisé en ce que** l'étape b) est réalisée par incubation pendant 30 à 120 minutes à une température de 20 à 25°C dans un milieu comprenant 0,5 à 2% (v/v) de polyoxyéthylène-p-t-octylphénol (Triton X-100, N° CAS 9002-93-1).

5. Procédé selon l'une quelconque des revendications 1 à 4, **caractérisé en ce que** le tampon utilisé lors de l'étape d) est un tampon trishydroxyméthylaminométhane (TRIS) à une concentration de 15 à 25 mM, un pH de 7,5 à 8,5 et une conductivité de 5 à 15 mS/cm.

6. Procédé selon l'une quelconque des revendications 1 à 5, **caractérisé en ce que** l'étape e) comprend en outre une filtration préalable à travers un filtre en profondeur comprenant des fibres de cellulose, de la terre de diatomée et une résine chargée négativement ou une membrane de polyéthersulfone d'une porosité de 0,22 µm fonctionnalisée par des groupements SO₃⁻.

7. Procédé selon l'une quelconque des revendications 1 à 6, **caractérisé en ce qu'**il comprend en outre une étape d'ultrafiltration et/ou de diafiltration.

8. Procédé selon l'une quelconque des revendications 1 à 7, **caractérisé en ce qu'**il est mis en oeuvre sur un surnageant de culture d'un clone producteur de l'anticorps monoclonal ou de la protéine de fusion entre le fragment Fc d'un anticorps et un second polypeptide.

9. Procédé selon l'une quelconque des revendications 1 à 8, pour la purification d'un anticorps monoclonal.

10. Procédé selon la revendication 9, **caractérisé en ce que** l'anticorps est dirigé contre l'un des antigènes suivants : Rhésus D, CD2, CD3, CD4, CD19, CD20, CD22, CD25, CD30, CD33, CD40, CD51 (Integrin alpha-V), CD52, CD80, CTLA-4 (CD152), SLAMF7 (CD319), Her2/neu, EGFR, EPCAM, CCR4, CEA, FR-alpha, GD2, GD3, HLA-DR, IGF1R (CD221), phosphatidylserine, TRAIL-R1, TRAIL-R2, antigènes de *Clostridium difficile*, antigènes de *Staphylococcus aureus*, antigènes du cytomégalovirus, antigènes *d'Escherichia coli*, antigènes du virus respiratoire syncytial, antigènes du virus de l'hépatite B, antigènes du virus Influenza A, antigènes de *Pseudomonas aeruginosa* sérotype IATS O11, antigènes du virus de la rage, ou phosphatidylserine.

## Patentansprüche

1. Verfahren zur Reinigung eines monoklonalen Antikörpers oder eines Fusionsproteins zwischen dem Fragment Fc eines Antikörpers und eines zweiten Polypeptids, umfassend:
a) einen Affinitätschromatographieschritt auf einem Harz mit einem vernetzten Methacrylatpolymergel als Matrix, auf dem das Protein A gepfropft ist,
b) einen viralen Inaktivierungsschritt,
c) einen Kationenaustauschchromatographieschritt auf einem Harz mit einem vernetzten Agarosegel als Matrix, auf dem Sulfonatgruppen (-SO₃-) über Spacerarme auf der Basis von Dextran gepfropft sind,
d) einen Anionenaustauschchromatographieschritt auf einer hydrophilen Polyethersulfonmembran, beschichtet mit einem vernetzten Polymer, auf dem quaternäre Amingruppen (Q) gepfropft sind, und
e) einen Nanofiltrationsschritt mit einem Filter mit einer doppelten Polyethersulfonmembran mit einer Porosität von zirka 20 nm,
**dadurch gekennzeichnet, dass** der in Schritt a) zum Eluieren des Antikörpers verwendete Elutionspuffer ein verwendeter Formiatpuffer mit einer Molarität von 5 bis 10 mM und einem pH zwischen 2,6 und 3,6 inklusive ist.

2. Verfahren nach Anspruch 1, **dadurch gekennzeichnet, dass** das vernetzte Methacrylatpolymergel, auf dem in Schritt a) verwendetes Protein A gepfropft ist, in Form von Kugeln mit einem durchschnittlichen Durchmesser zwischen 30 und 60 µm, in vorteilhafter Weise zwischen 40 und 50 µm, inklusive vorliegt.

3. Verfahren nach Anspruch 1 oder Anspruch 2, **dadurch gekennzeichnet, dass** der Schritt a) einen Unterschritt des Waschens des Harzes mit einer Salzlösung umfasst, umfassend eine NaCl-Konzentration von mindestens 1 M.

4. Verfahren nach einem der Ansprüche 1 bis 3, **dadurch gekennzeichnet, dass** der Schritt b) durch Inkubation während 30 bis 120 Minuten bei einer Temperatur von 20 bis 25 °C in einem Milieu, welches 0,5 bis 2 % (v/v) Polyoxyethylen-p-t-octylphenol (Triton X-100, N° CAS 9002-93-1) umfasst, durchgeführt wird.

5. Verfahren nach einem der Ansprüche 1 bis 4, **dadurch gekennzeichnet, dass** der bei Schritt d) verwendete Puffer ein Trishydroxymethylaminmethanpuffer (TRIS) mit einer Konzentration von 15 bis 25 mM, einem pH von 7,5 bis 8,5 und einer Leitfähigkeit von 5 bis 15 mS/cm ist.

6. Verfahren nach einem der Ansprüche 1 bis 5, **dadurch gekennzeichnet, dass** der Schritt e) ferner eine vorherige Filtration durch einen Tiefenfilter umfasst, umfassend Zellulosefasern, Diatomeenerde und ein negativ geladenes Harz oder eine Polyethersulfonmembran mit einer Porosität von 0,22 µm, funktionalisiert durch SO₃⁻-Gruppen.

7. Verfahren nach einem der Ansprüche 1 bis 6, **dadurch gekennzeichnet, dass** es ferner einen Ultrafiltrations- und/oder Diafiltrationsschritt umfasst.

8. Verfahren nach einem der Ansprüche 1 bis 7, **dadurch gekennzeichnet, dass** es auf einem Kulturüberstand eines Klons, der den monoklonalen Antikörper produziert, oder des Fusionsproteins zwischen dem Fragment Fc eines Antikörpers und eines zweiten Polypeptids durchgeführt wird.

9. Verfahren nach einem der Ansprüche 1 bis 8 für die Reinigung eines monoklonalen Antikörpers.

10. Verfahren nach Anspruch 9, **dadurch gekennzeichnet, dass** der Antikörper gegen eins der folgenden Antigene gerichtet ist: Rhesus D, CD2, CD3, CD4, CD19, CD20, CD22, CD25, CD30, CD33, CD40, CD51 (Integrin Alpha-V), CD52, CD80, CTLA-4 (CD152), SLAMF7 (CD319), Her2/neu, EGFR, EPCAM, CCR4, CEA, FR-Alpha, GD2, GD3, HLA-DR, IGF1R (CD221), Phosphatidylserin, TRAIL-R1, TRAIL-R2, Antigene von *Clostridium difficile*, Antigene von *Staphylococcus aureus*, Antigene des Cytomegalovirus, Antigene von *Escherichia coli*, Antigene des Respiratory-Syncytial-Virus, Antigene des Hepatitis B-Virus, Antigene des Influenza A-Virus, Antigene von *Pseudomonas aeruginosa* Serotyp IATS O11, Antigene des Tollwutvirus, oder Phosphatidylserin.

## Claims

1. Method for purification of a monoclonal antibody or a fusion protein between the Fc fragment of an antibody and a second polypeptide, comprising:
a) an affinity chromatography step on a resin having a cross-linked methacrylate polymer gel as a matrix, on which protein A is grafted,
b) a viral inactivation step,
c) a cation-exchange chromatography step on a resin having a cross-linked agarose gel as a matrix, on which sulfonate groups (-SO₃-) are grafted via dextran-based spacer arms,
d) an anion-exchange chromatography step on a hydrophilic polyethersulfone membrane coated with a cross-linked polymer on which quaternary amine groups (Q) are grafted, and
e) a nanofiltration step with a filter having a polyethersulfone double membrane having a pore size of about 20 nm,
**characterized in that** the elution buffer used in step a) to elute the antibody is a formate buffer used at a molarity of 5 to 10 mM and at a pH of between 2.6 and 3.6.

2. Method according to claim 1, **characterized in that** the cross-linked methacrylate polymer gel on which protein A is grafted used in step a) is in the form of beads having an average diameter of between 30 and 60 µm, advantageously of between 40 and 50 µm.

3. Method according to claim 1 or claim 2, **characterized in that** step a) comprises a sub-step of washing the resin with a saline solution comprising a NaCl concentration of at least 1 M.

4. Method according to any one of claims 1 to 3, **characterized in that** step b) is carried out by incubation for 30 to 120 minutes at a temperature of 20 to 25°C in a medium comprising 0.5 to 2% (v/v) polyoxyethylene-p-t-octylphenol (Triton X-100, CAS no. 9002-93-1).

5. Method according to any one of claims 1 to 4, **characterized in that** the buffer used during step d) is a trishydroxymethylaminomethane (TRIS) buffer at a concentration of 15 to 25 mM, a pH of 7.5 to 8.5 and a conductivity of 5 to 15 mS/cm.

6. Method according to any one of claims 1 to 5, **characterized in that** step e) further comprises prior filtration through a depth filter comprising cellulose fibers, diatomaceous earth and a negatively-charged resin or a polyethersulfone membrane having a pore size of 0.22 µm functionalized by SO₃⁻-groups.

7. Method according to any one of claims 1 to 6, **characterized in that** it further comprises a step of ultrafiltration and/or of diafiltration.

8. Method according to any one of claims 1 to 7, **characterized in that** it is implemented on a culture supernatant of a clone producing the monoclonal antibody or the fusion protein between the Fc fragment of an antibody and a second polypeptide.

9. Method according to any one of claims 1 to 8, for the purification of a monoclonal antibody.

10. Method according to claim 9, **characterized in that** the antibody is directed against one of the following antigens: Rhesus D, CD2, CD3, CD4, CD19, CD20, CD22, CD25, CD30, CD33, CD40, CD51 (Integrin alpha-V), CD52, CD80, CTLA-4 (CD152), SLAMF7 (CD319), Her2/neu, EGFR, EPCAM, CCR4, CEA, FR-alpha, GD2, GD3, HLA-DR, IGF1R (CD221), phosphatidylserine, TRAIL-R1, TRAIL-R2, *Clostridium difficile* antigens, *Staphylococcus aureus* antigens, cytomegalovirus antigens, *Escherichia coli* antigens, respiratory syncytial virus antigens, hepatitis B virus antigens, Influenza A virus antigens, *Pseudomonas aeruginosa* serotype IATS O11 antigens, rabies virus antigens, or phosphatidylserine.
